# EUROPEAN PATENT APPLICATION

(11) **EP 4 768 080 A2**
(43) Date of publication of application: **01.07.2026**
(21) Application number: 26174882.6
(22) Date of filing: 04.10.2023
(51) Int. Cl.: A61P 25/00

(54) **METHOD OF TREATING NEONATES WITH IGF-1 COMPLEX**

(30) Priority: 04.10.2022 US 202263378267 P; 29.09.2023 GB 202315046
(62) Divisional of application: 23783842.0
(71) Applicant: OAK HILL BIO LIMITED, Altrincham Cheshire WA14 2DT (GB)
(72) Inventor: CAREY, Galen John, Massachusetts, MA 02478 (US); BARTON, Norman, Pheonix, 21131 (US); LEY, David Charles Reginald, 221 85 Lund (SE); EKSTRÖM, Claes Per, 221 25 Lund (SE); GRAM, Magnus Göran, 221 85 Lund (SE); ORTENLÖF, Niklas Emanuel, 221 85 Lund (SE)
(74) Representative: STERLING IP LTD

(57) **Abstract**

The present disclosure relates to treatment of subpopulation of neonates for reducing the risk of IVH and/or severe IVH, such as grade 3 or 4 IVH by administering a therapeutic amount of a composition comprising IGF-1 and an IGF binding protein (such as IGFBP-3), for example as a complex, at a dose of 200-500µg/Kg/day. In a further aspect there is provided stabilisation of vital functions when hyperosmolarity occurs and/or protection from circulatory collapse.

## Description

The present disclosure relates to treatment of subpopulation of neonates for reducing the risk of IVH and/or severe IVH, such as grade 3 or 4 IVH. In a further aspect there is provided stabilise of vital functions when hyperosmolarity occurs and/or protection from circulatory collapse.

### BACKGROUND

The preterm infants as a patient population are some of the most delicate, vulnerable and difficult to treat. Some of these infants have only 4 tablespoons of blood in their whole body. This leads to large variations in parameters, such as blood pressure, measured in this population. It is also very difficult to do clinical trials in this patient population, for example sampling and administration is not easy, even a saline drip has the potential to cause a brain haemorrhage because of an inability of these infants to autoregulate.

Many drugs are not licensed for use in these infants and the dose has not been established adequately. What is more, reaching statical significance is not straightforward because of the variability between individual patients.

Fluctuations in blood flow and pressure are common. Low pressure can cause constriction in vital organs such as the kidney. Increases in cerebral blood flow can cause intraventricular haemorrhage (IVH) and death. Unfortunately, the latter is fairly common in preterm infants, especially those born extremely premature, i.e. born in gestational week 23-27.

Neonates with a low gestation age (in particular those born at 23, 24, 25 and 26 weeks of gestation) and neonates with low APGAR score, a measure used to assess the stress/trauma status of the neonate, are particularly susceptible to IVH.

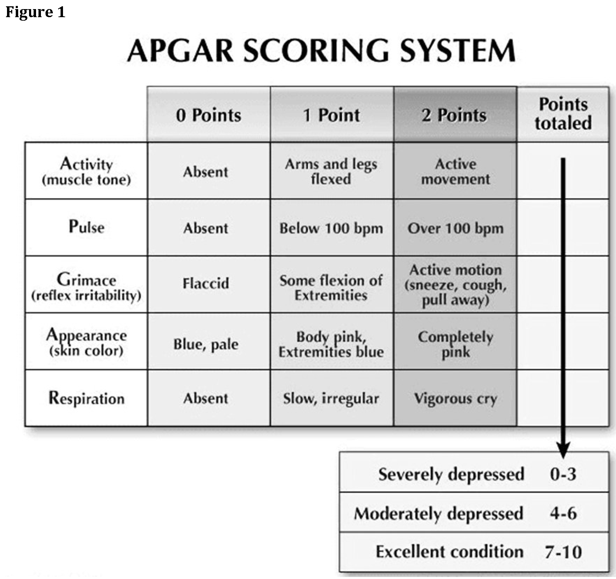

APGAR scores: Activity (muscle tone) in a range 0-2, Pulse in a range 0-2, Grimace (reflex irritability) in a range 0-2, Appearance (skin colour) in the range 0-2, and Respiration in the range 0-2, see Figure 1. Total scores in the range 7-10 mean an infant is in excellent condition. Total scores in the range 4-6 mean the infant is moderately depressed. Total score in the range 0-3 means the infant is severely depressed.

Instability in blood pressure and/or respiration problems, kidney function problems seem to contribute to stressing the body, which in turn may lead to an increased risk of IVH. This may explain why neonates with a low APGAR are more prone to IVH.

What is more the medical interventions used to treat these very sick neonates can also cause problems, for example as explained above a saline drip has the potential to cause IVH because of the inability of these infants to autoregulate. Resuscitation can also have a negative impact on IVH, in particular if the infant is overtreated to have pink skin tone this may increase the risk of IVH. In fact, it has been found that gentle resuscitation is better. Inadequate urine excretion may require treatment with a vasopressor to raise blood pressure and/or a diuretic. These treatments can inadvertently contribute to IVH.

It would be useful to understand in more detail the mechanisms associated with IVH in this patient population and translate this into beneficial treatments to minimise the incidences or severity of IVH because at the present time none exist.

Rodent studies have shown that systemic insulin-like growth factor 1 (IGF-1) can bind to, and translocate through, the choroid plexus (CP) into cerebrospinal fluid. However, previous studies have inferred that the transfer of systemic IGF-1 across the blood brain barrier is restricted, presenting a potential limitation for beneficial treatment effects within the brain.

A detailed characterization by the inventors has shown that IGF-1/IGF binding protein 3 (IGF-1/IGFBP-3) activates the IGF-1 receptor (IGF-1R) and downstream signalling pathway at the CP in the immature brain of the preterm rabbit pup. This gives confidence that IGF-1 can act at the site where it is needed.

What is more detailed analysis by the inventors of the human neonate clinical data shows that prevention of IVH in up to 60% of neonate receiving IGF-1/IGFBP-3 may be achievable, in low gestational age infants. This may be as much as 22% more IVH free infants in the treated population in comparison to those infants who simply received standard of care. In other instances, the severity of the IVH is reduced in the treated population i.e. grade 3 and grade 4 instances of IVH are reduced.

In addition, the treated infants are more robust, for example blood pressure stabilised, ability to urinate is stabilised (for example no need to administer a vasopressor and/or diuretic), and respiratory parameters are improved, for example as evidenced by the measurement of respiratory gases. This may in turn contribute to a lower propensity towards IVH.

Thus, neonates with a low APGAR score who also have an increased risk of IVH will also benefit from IGF-1/IGFBP-3 treatment because it helps stabilise the functions that are depressed.

Abnormalities in glucose homeostasis, including insulin resistance, are also common in these infants. This may cause changes in plasma osmolarity (that can be sudden) that leads to systemic effects and disfunctions in preterm infants, such as circulatory collapse and/or IVH.

The present inventors have performed work in preterm animal models to establish the mechanisms involved in circulatory collapse or acute metabolic syndrome and IVH.

One animal model employed for this purpose by the inventors was the rabbit pup model where 50% glycerol administered systemically in preterm animals was used to generate hyperosmolarity. This in turn leads to disturbance of metabolic systems, changes in glucose levels and significant changes in blood flow including mean arterial pressure (in particular more variable and/or higher pressure) and increased cerebral blood flow is observed which in turn causes bleeds to occur in the brain. Thus, this model simulates circulatory collapse and IVH.

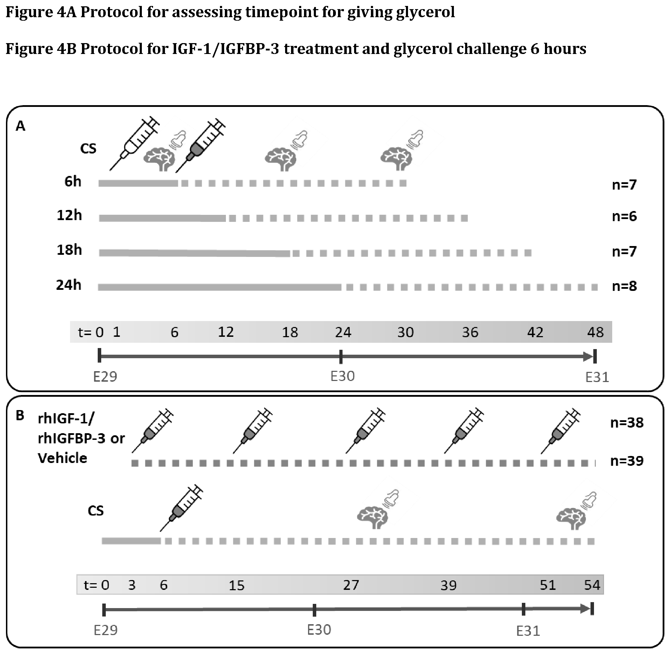

The rabbit pup glycerol-induced IVH model disclosed herein, administers treatment (with IGF-1/IGFBP-3) 3 hours from birth (continuing in a regimen every 12 hours up to experimental end-point) and glycerol at 6 hours, as per the schedule shown in Figure 4B.

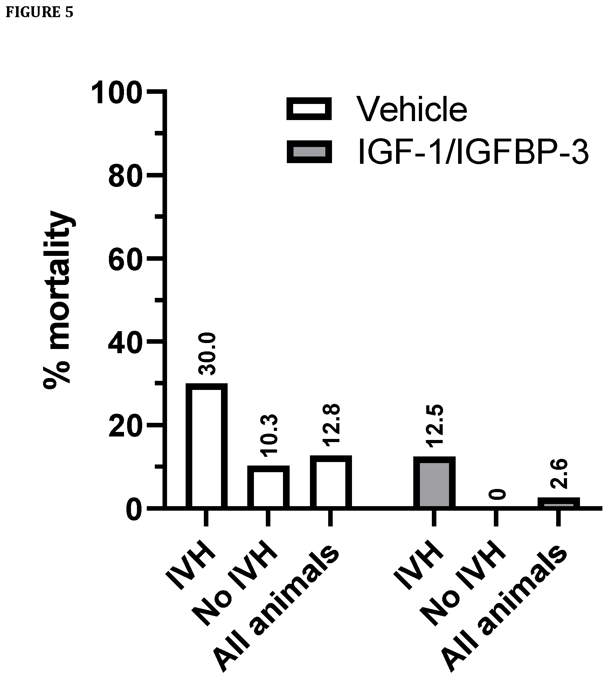

Where IVH was induced there was more than a 50% reduction in the mortality of IGF-1/IGFBP-3 treated preterm rabbit pups in comparison to untreated pups, see Figure 5.

Interestingly, 10% of untreated animal died of systemic effects with no IVH, likely due to vascular collapse or acute metabolic syndrome. In contrast **zero** IGF-1/IGFBP-3 treated animals died with no IVH.

Thus, IGF-1/IGFBP-3 seems to have a function in the regulation of blood flow and/or pressure. The observed mortality benefit is likely to come from the ability of treated animals to stabilise the same in response to a sudden change in plasma osmolarity.

A small amount of protection from induced IVH was seen in the IGF-1/IGFBP-3 treated group at 24 hours, 21% vs 25.6% in the untreated group, see Table 3 below.

The inventors know from other work (not shown) that the effects of administering IGF-1/IGFBP-3 takes about 24 hours to take effect. Thus, a glycerol challenge at 6 hours, induces IVH before the treatment has really had time to be effective. It is hypothesised that a more pronounced protection against induced IVH could be provided when the glycerol challenge is administered later, for example at 18 hours because no further incidences of IVH were seen in the treated group at 48 hours. In contrast IVH in the untreated rabbit pups increased from 25.6% at 24 hours to 28.2% at 48 hours, see table 3 in Example 2.

In summary the present inventors have shown that IGF-1 can stimulate signalling across the blood brain barrier after systemic administration, and that the IGF-1 in the brain is able to reduce the incidences and/or severity of IVH in the very preterm infants, in particular low gestational age preterm infants, such as 23 to 27 weeks, in particular 23 to 26 weeks, especially 23 to 25 and/or 26 weeks. The treatment is also likely to treat low birth weight babies and infants with a low APGAR score, and combinations of these patient populations.

The treatment also seems to help stabilise fluctuations in hyperosmolarity.

Treatment may also assist infants with autoregulation.

Surprisingly the IGF-1 penetrates the premature brain, in particular without use of IGF-1R. IGF-1/IGFBP-3 is also hypothesised by the inventors to be beneficial to one or more of the following: brain plasticity, ion channel regulation, membrane permeability, and/or fluid "absorptions". This may facilitate the preterm to minimise damage caused by an IVH.

### SUMMARY OF THE DICLOSURE

1. A method of preventing intraventricular haemorrhage (IVH) in a preterm infant born at a low gestational age (for example 23, 24, 25 or 26 weeks, such as 23, 24 or 25 i.e. 23 to 25 weeks) by administering a therapeutic amount of a composition comprising IGF-1 and an IGF binding protein (such as IGFBP-3), for example as a complex, at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, and wherein after exclusion of baseline assessment timepoint more than 50% of those infants receiving treatment are IVH free.
2. A composition for use in preventing intraventricular haemorrhage (IVH) in a preterm infant born at a low gestational age (for example 23, 24, 25 or 26 weeks, such as 23, 24 or 25 i.e. 23 to 25 weeks), comprising IGF-1 and an IGF binding protein (such as IGFBP-3), for example as a complex, wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, and wherein after exclusion of baseline assessment timepoint more than 50% of those infants receiving treatment are IVH free.
3. Use of a composition comprising IGF-1 and an IGF binding protein (such as IGFBP-3), for example as a complex, in the manufacture of a medicament for preventing intraventricular haemorrhage (IVH) in a preterm infant born at a low gestational age (for example 23, 24, 25 or 26 weeks, such as 23, 24 or 25 i.e. 23 to 25 weeks), wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, and wherein after exclusion of baseline assessment timepoint more than 50% of those infants receiving treatment are IVH free.
4. A method, composition or use according to any one of paragraphs 1 to 3, wherein 51, 52, 53, 54, 55, 56, 57, 58, 59, 60, 61, 62, 63, 64, 65% or more of the treated patient population are free from IVH.
5. A method, composition or use according to any one of paragraphs 1 to 4, wherein at least 70% of the treated population, for example 71, 72, 73, 74, 75, 76, 77, 78, 79, 80% or more of the treated population is below grade 2 (i.e. 0 or 1) on the IVH scale, for example by VOLPE method and/or maximum score method.
6. A method, composition or use according to any one of paragraphs 1 to 5, wherein at least 85% of the treated population, for example 85, 86, 87, 87.5% or more of the treated population is below grade 3 (i.e. 0, 1 or 2) on the IVH scale, for example by VOLPE method and/or maximum score method.
7. A method of preventing intraventricular haemorrhage (IVH) grade 3 or 4 in a preterm infant born at a low gestational age (for example 23, 24, 25 or 26 weeks, such as 26 weeks), by administering a therapeutic amount of a composition comprising IGF-1 and an IGF binding protein (such as IGFBP-3), for example as a complex, at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, wherein after exclusion of the baseline timepoint 15% or less of those infants receiving treatment have grade 3 or 4 IVH, such as 12. 5%, 12%, 11%, 10%, 9%, 8%, 7%, 6.5%, 6.25% or less, in particular 10% or less.
8. A composition for use in preventing intraventricular haemorrhage (IVH) grade 3 or 4 in a preterm infant born at a low gestational age (for example 23, 24, 25 or 26 weeks, such as 26 weeks), comprising IGF-1 and an IGF binding protein (such as IGFBP-3), for example as a complex, wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, wherein after exclusion of the baseline timepoint 15% or less of those infants receiving treatment have grade 3 or 4 IVH, such as 12. 5%, 12%, 11%, 10%, 9%, 8%, 7%, 6.5%, 6.25% or less, in particular 10% or less.
9. Use of a composition comprising IGF-1 and an IGF binding protein (such as IGFBP-3), for example as a complex, in the manufacture of a medicament for preventing intraventricular haemorrhage (IVH) grade 3 or 4 in a preterm infant born at a low gestational age (for example 23, 24, 25 or 26 weeks, such as 26 weeks), wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, wherein after exclusion of the baseline timepoint 15% or less of those infants receiving treatment have grade 3 or 4 IVH, such as 12. 5%, 12%, 11%, 10%, 9%, 8%, 7%, 6.5%, 6.25% or less, in particular 10% or less.
10. A method of preventing intraventricular haemorrhage (IVH) or preventing grade 3 or 4 IVH in a preterm infant with an APGAR score below 7 by administering a therapeutic amount of a composition comprising IGF-1 and an IGF binding protein (such as IGFBP-3), for example as complex, at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, and wherein after exclusion of baseline assessment timepoint a higher percentage of infants receiving treatment are IVH free or grade 3 or 4 free, in comparison to comparable untreated preterm infants.
11. A composition for use in preventing intraventricular haemorrhage (IVH) or preventing grade 3 or 4 IVH in a preterm infant with an APGAR score below 7, comprising IGF-1 and an IGF binding protein (such as IGFBP-3), for example as complex, wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, and wherein after exclusion of baseline assessment timepoint a higher percentage of infants receiving treatment are IVH free or grade 3 or 4 free, in comparison to comparable untreated preterm infants.
12. Use of a composition comprising IGF-1 and an IGF binding protein (such as IGFBP-3), for example as complex, in the manufacture of a medicament for preventing intraventricular haemorrhage (IVH) or preventing grade 3 or 4 IVH in a preterm infant with an APGAR score below 7, wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period, and wherein after exclusion of baseline assessment timepoint a higher percentage of infants receiving treatment are IVH free or grade 3 or 4 free, in comparison to comparable untreated preterm infants.
13. A method of stabilising pathological fluctuations in blood osmolarity in a preterm infant (for example a low gestational age preterm) by administering a therapeutic amount of a composition comprising IGF-1 and an IGF binding protein (such as IGFBP-3), for example as complex, at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period.
14. A composition for use in stabilising pathological fluctuations in blood osmolarity in a preterm infant (for example a low gestational age preterm), comprising IGF-1 and an IGF binding protein (such as IGFBP-3), for example as complex, wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period.
15. Use of a composition comprising IGF-1 and an IGF binding protein (such as IGFBP-3), for example as complex, in the manufacture of a medicament for stabilising pathological fluctuations in blood osmolarity in a preterm infant (for example a low gestational age preterm), wherein the composition is administered at a dose of 200-500µg/Kg/day (such as 250, 300, 350, 400, 450 or 500) within 3 hours of birth for a period of at least 7 days, such that plasma levels of IGF-1 are maintained within the therapeutic range 28µg/L to 109µg/L during the treatment period.
16. A method, composition or use according to any preceding paragraph, wherein the incidences of IVH occurring and/or progressing are ameliorated in the time period 24 hours to 168 hours after initiations of treatment, such as 24 hours to 120 hours, including 48 to 120 hours.
17. A method, composition or use according to any one of the preceding paragraphs wherein a treated preterm has a mortality benefit.
18. A method, composition or use according to any one of the preceding paragraphs wherein the risk of IVH is reduced by at least 10%, for example 11, 12, 13, 14, 15, 16, 17, 18% or more by 48 hours (and longer such as at least 50, 60 or 72 hours).
19. A method, composition or use according to any one of the preceding paragraphs, wherein the severity of IVH is reduced, for example incidence of grade 3 and/or 4 is reduced.
20. A method, composition or use according to any one of the preceding paragraphs, wherein the severity of IVH grade 2 is reduced.
21. A method, composition or use according to any preceding paragraphs, wherein treatment is initiated within 24 hours from birth, for example within 30 minutes to 3 hours from birth.
22. A method, composition or use according to any preceding paragraph, wherein the treatment is administered subcutaneously and/or by infusion (such as intermittent or continuous infusion).
23. A method, composition or use according to any preceding paragraph, wherein the preterm infants are treated for at least 5 days, for example for at least 1 week, such as 2 to 6 weeks, such as 2, 3, 4, 5 or 6 weeks.
24. A method, composition or use according to any preceding paragraph, wherein treatment is continued until 32, 33 or 34 weeks gestational age.
25. A method, composition or use according to any preceding paragraph, wherein serum levels of IGF-1 are maintained within the range 28 to 109ng/mL.

In one embodiment germinal matrix haemorrhage (GMH) is reduced in the treated patient population in comparison to the untreated patient population.

In one embodiment periventricular haemorrhagic infarction (PVH) is reduced in the treated patient population in comparison to the untreated patient population.

In one embodiment post-haemorrhagic ventricular dilatation (PHVD), and/or white matter injury (WMI) is reduced (or minimised) in the treated patient population in comparison to the untreated patient population.

Thus in one embodiment treated neonates born in the range 23 to 25 weeks have a reduced incidence of IVH all levels including severe IVH.

In one embodiment treated neonates born at 26 weeks are protected from severe incidence of IVH such as grade 3 and/or grade 4.

In an independent aspect there is provided stabilisation of vital functions such as breathing, heart rate and blood pressure in the presence of hyperosmolarity, by administering a therapeutic amount of a composition comprising IGF-1 and an IGF binding protein (such as IGFBP-3), for example as a complex, at a dose of 200-500µg/Kg/day (such as 250, 300, 350, 400, 450 or 500, in particular 350-500µg/Kg/day), for example as described elsewhere herein.

### DETAILED DISCLOSURE

Treatment period as employed herein is the period after administration of the protein IGF-1/IGFBP (such as IGFBP-3) when said proteins becomes effective. This may be when the levels of circulating IGF-1 are within the therapeutic range, for example 28µg/L to 109µg/L in a human preterm. It may take in the region of 24 to 48 hours after administration of the first dose to fully reach the treatment period.

Advantageously, doses of at least 350µg/Kg/day ensure that the majority of neonates treated have circulating IGF-1 levels within the therapeutic range (28 to 109ng/mL).

Low gestational age preterm as employed herein refers to an infant of gestational age 27 weeks or less, for example 26 weeks or less, 25 weeks or less, 24 weeks or less, such as 26, 25, 24 and 23 weeks, in particular 23 to 25 weeks or 23 to 26 weeks.

'After exclusion of baseline assessment timepoint' as employed herein is intended to exclude those neonates who have IVH at timepoint zero.

"Comprising" in the context of the present specification is intended to mean "including". Where technically appropriate, embodiments of the invention may be combined.

Embodiments are described herein as comprising certain features/elements. The disclosure also extends to separate embodiments consisting or consisting essentially of said features/elements.

Technical references such as patents and applications are incorporated herein by reference.

Any embodiments specifically and explicitly recited herein may form the basis of a disclaimer either alone or in combination with one or more further embodiments.

Values (such as numerical values and/or variables such as R1 etc) in the examples may be extracted from the a specific example and combined with a disclosure (such as a generic disclosure) from the description without incorporating other features of the example.

The present specification claims priority from US63/378,267 filed 4 October 2022 and GB2315046.9 filed on 29 September 2023, both incorporated herein by reference. These specifications may be used as basis for corrections in the present specification.

The background contains useful technical information and can be used as basis for amendments.

The invention will now be described with reference to the following examples, which are merely illustrative and should not be construed as limiting the scope of the present invention.

### SUMMARY OF THE FIGURES

- **Figure 1**: Shows a diagrammatic representation of the APGAR scoring system.
- **Figure 2**: Shows plasma osmolarity in preterm rabbit pups administered glycerol.
- **Figure 3**: Shows the effects of glycerol administration heart rate, oxygen saturation and breathing rate on preterm rabbit pups.
- **Figure 4A**: Shows a diagrammatic representation of the protocol for assessing which timepoints are suitable for administering a glycerol challenge.
- **Figure 4B**: Shows a diagrammatic representation of the protocol for administering IGF-1/IGFBP-3 treatment in conjunction with glycerol challenge at 6 hours.
- **Figure 5**: Shows percentage mortality (all causes) for preterm rabbit pups treated under protocol shown in Figure 4B.
- **Figure 6A**: Shows a preterm rabbit pup brain without IVH.
- **Figure 6B**: Shows a preterm rabbit pup brain with IVH.
- **Figure 7A**: Shows serum levels of IGF-1 up to 48 hours
- **Figure 7B**: Shows serum levels of IGF-1 at 48 hours for preterm rabbit pups with no IVH and those with IVH.
- **Figure 7C**: Shows levels of recombinant human IGF-1 and IGFBP-3 in preterm rabbit pups.
- **Figure 8**: Shows representative lightsheet microscopy 3D image illustrating Alexa Fluor-647 labeled IGF-1/IGFBP3 (yellow) in preterm rabbit brain 5 hours after subcutaneous administration of Alexa Flour-647 labeled IGF 1/IGFBP-3. For visualization, autofluorescence (displayed in red) is included
- **Figure 9**: Shows representative lightsheet microscopy 2D images illustrating Alexa Fluor-647 labeled IGF-1/IGFBP3 (yellow) in preterm rabbit brain 5 hours after subcutaneous administration of Alexa
- **Figure 10**: Shows representative confocal and transmission electron microscopy images illustrating immunolabeling against IGF-1/IGFBP-3, choroid plexus (left) and subfornical organ (middle) red, choroid plexus (right) white and grey arrows, in preterm rabbit brain 5 hours after subcutaneous administration
- **Figure 11**: Westernblot of p.ERK and p.PKB of choroid plexus in preterm rabbit brain 5 hours after subcutaneous administration of IGF-1/IGFBP-3

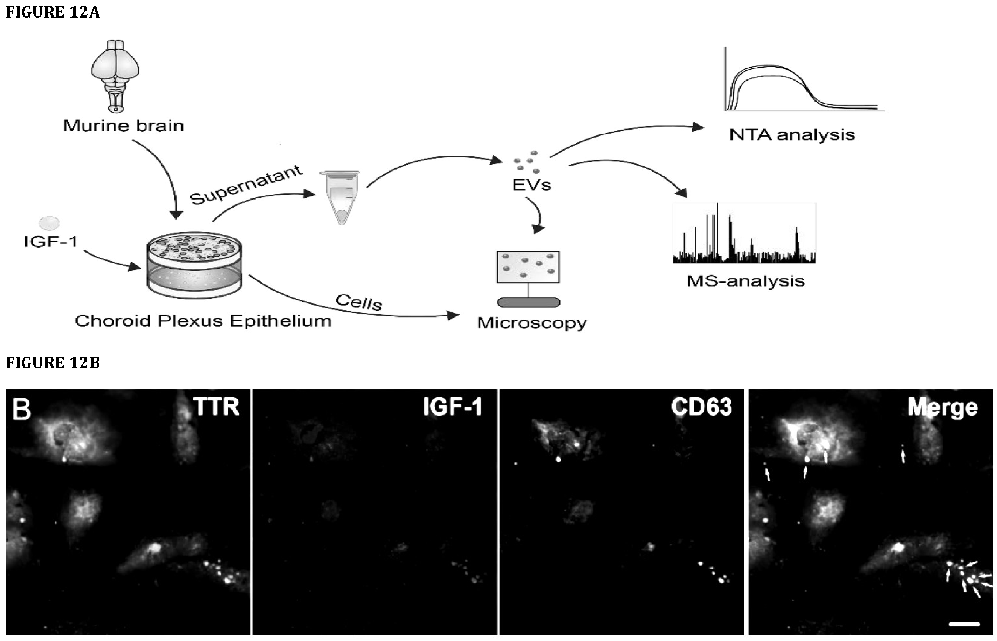

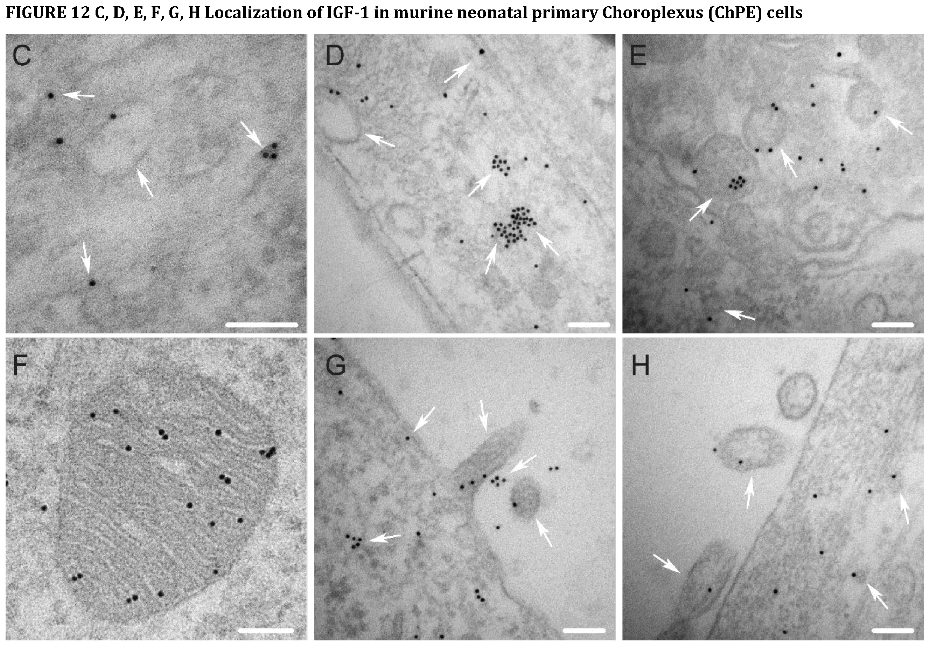

- **Figure 12**: **A.** Schematic illustration of the in vitro study outline. **B.** Representative confocal microscopy images illustrating labeling of TTR (green, **B,** far left and far right), IGF-1 (magenta, **B,** middle-left and far right), and CD63 (yellow, **B** middle-right, and far right) in neonatal primary murine ChPE cells cultured in the transwell system. Co-localization of IGF-1 with the late endosomal marker CD63 is displayed by white arrows in the merged image (B, far right). Scale bar represents 20 µm and is representative for all images in **B. C-H.** Representative TEM images illustrating immunogold labeling of flotillin-2(**C**) and IGF-1 **(D-H)** in neonatal primary murine ChPE cells cultured in the transwell system. White arrows display immunogold labeling in membrane enclosed vesicles. Scale bars in **C-H** represents 100 nm. Confocal microscopy and TEM images are collected from 3 independent experiments.
- **Figure 13A&B**: Shows representative TEM image of purified neonatal primary murine CPE cell derived EVs immunogold labeled with flotillin-2 **(A)** and IGF-1 **(B).**
- **Figure 13C&D**: Shows NTA quantification **(C),** and size distribution **(D)** of EVs secreted into the apical supernatant of the ChPE cells following exposure to hIGF-1 (40, 100 and 250 ng/ml) for 24 hours. Data are from four independent experiments with N=4-6 at respective experiment. Data are presented as means ± SD. Differences between 40, 100 and 250 ng/ml hIGF-1 vs. Control was analyzed using one-way ANOVA with post hoc Tukey test for multiple comparisons of means, *P≤0.01.
- **Figure 14A&B**: Shows **A.** Volcano plot of the proteome in EVs derived from IGF-1 exposed (40 ng/ml) ChPE cells compared with Control cells. **B.** Significant changes in abundance of proteins identified in EVs derived from ChPE cells, with the significance criteria fold-change ≥ 1.5 and ≤ -1.5, and adjusted p-value, Benjamini-Hochberg-corrected p-value ≤ 0.05, following hIGF-1 (40 ng/ml) stimulation.
- **Figure 14C&D**: Shows GO biological processes terms based on David analysis for individual proteins are displayed. Metascape analysis of enriched (C) and suppressed **(D)** proteins identified in EVs derived from ChPE cells with the significance criteria log fold-change ≥ 1 and ≤ -1, and p-value ≤ 0.05, following hIGF-1 (40 ng/ml) stimulation.
- **Figure 15A-C**: Shows **A.** Volcano plot of the proteome in preterm piglet CSF derived EVs exposed to hIGF-1 compared to vehicle infused piglets. **B.** Metascape analysis of suppressed proteins identified in EVs derived from preterm piglet CSF upon IGF-1 treatment with the significance criteria log fold-change ≥ 1 and ≤ -1, and p-value ≤ 0.05. C. Venn diagram displaying overlap of proteins derived from ChPE cell supernatant EVs (purple, N = 12) or preterm piglet CSF EVs (green, N = 16).
- **Figure 15D**: Shows reactome gene sets

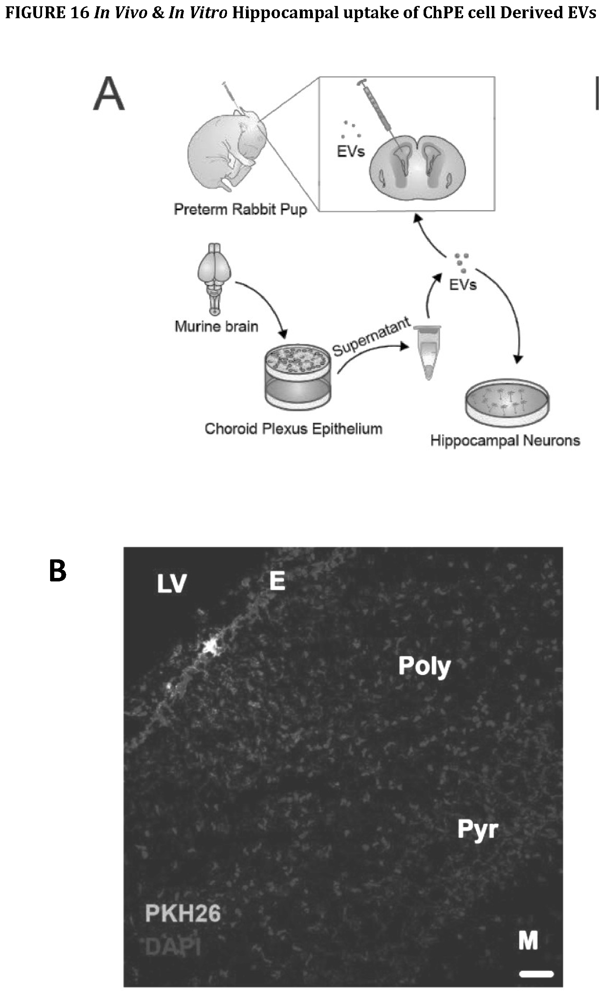

- **Figure 16A-E**: Shows **A** Schematic illustration of the experimental outline. **B-D**. Representative confocal microscopy images illustrating presence of ChPE cell derived EVs stained with the dye PKH26 (red) in the hippocampus of the preterm rabbit pup brain following i.c.v injections. **B** and **C**. Representative confocal microscopy images illustrating EVs beyond the ependymal layer in the polymorph layer. **D.** Representative confocal microscopy images illustrating EVs in deep structures of the CA2-CA3 regions of the hippocampus proper in the pyramidal layer and further in the molecular layer. Representative images are collected from N = 8. **E.** Scale bars in **B-D** represents 50 µm. GAP-43 labeled rat hippocampal neurons (red) incubated with ChPE cell derived EVs stained with PKH27 (green). Scale bar in E represents 25 µm.
- **Figure 16F**: Shows quantification of green signal per number of nuclei in hippocampal neurons exposed to EVs derived from hIGF-1 stimulated ChPE cells. Data is presented as means ± SD (N = 12). Differences between groups were analyzed using Student's t-test. E, ependymal layer; poly, polymorph layer; pyr, pyramidal layer; M, molecular layer; LV, lateral ventricle.
- **Figure 17**: Shows a diagrammatic representation of a suggested model of IGF-1 transpor The ChP constitutes the barrier between the periphery and the CSF of the brain. This highly secretory organ increases the secretion of EVs upon blood borne IGF-1 stimulation. The blood-borne IGF-1 is transported through the blood-CSF barrier, encapsulated in EVs secreted from the ChP, ultimately reaching the hippocampus in the immature brain.

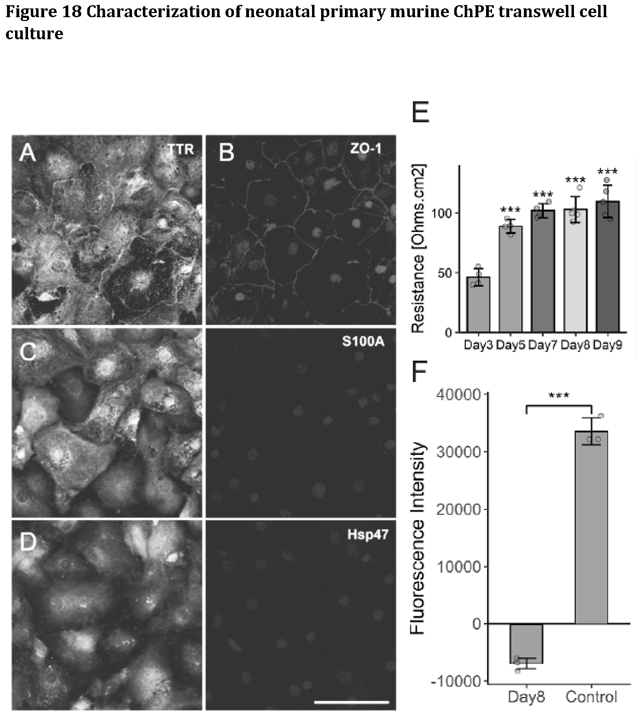

- **Figure 18**: Shows characterization of neonatal primary murine ChPE transwell cell culture model. **A-D.** Representative confocal microscopy images illustrating the expression of TTR (grey areas, A, C, D, left), ZO-1 (grey lines, B, right), S100A4 (grey areas, C, right) and Hsp47 (grey areas, D, right). Scale bar in D represents 100 µm and is representative for A-D. **E.** TEER measurement of the ChPE cell culture in the transwell system. Representative data are presented as means ± SD (N=4-5/group). Differences in TEER measurement, as compared to day 3, were analyzed using one-way ANOVA with post hoc Tukey for multiple comparisons of means, ***P≤0.001. **F.** Transcytosis assay of ChPE cells in the transwell system at day 8 of culture. Representative data are presented as means ± SD (N = 3-4/group). Differences vs. Control were analyzed using Student's t-test, ***P≤0.001.
- **Figure 19**: Shows localization of IGF-1R in neonatal primary murine ChPE cells. Representative confocal microscopy images illustrating labeling of IGF-1R (grey dots, left and right), and CD63 (white dots, middle, and right) in neonatal primary murine CPE cells cultured in a transwell system. Co-localization is displayed by white arrows in the merged image (right) Scale bar represents 20 µm and is representative for all images.
- **Figure 20**: Shows ultrasound guided i.c.v. injection. EVs prepared from hIGF-1 stimulated or control neonatal primary murine ChPE cells in vitro were stained with PKH26, and subsequently i.c.v. injected (ultrasound guided) into the lateral ventricles of non-sedated preterm rabbit pups. **A.** Injected needle is located in the lateral ventricle. **B.** Injection of fluid into the lateral ventricle. **C.** Needle withdrawal. LV, lateral ventricle.
- **Figure 21**: Shows investigated area uptake of PKH27 dye. **A and B.** Representative confocal microscopy images illustrating i.c.v. injected PKH26 dye (indicated by arrows) in the investigated area of the brain in the preterm rabbit.
- **Figure 22**: Shows hippocampal neuron uptake of PKH67 dye in vitro. **A and B.** Representative confocal microscopy images displaying visualization of PKH67 (white areas, **A)** and GAP-43 (light grey areas, **A-B)** in hippocampal neuron in vitro. Hippocampal neurons were incubated with either PKH67 dye **(A)** or with PBS **(B).** Scale bar in B display 50 µm and is representative for A and B.

### EXAMPLES

### Example 1 Neonate Phase II study findings relative to IVH

A phase II trial in which infants less than 28 weeks gestational age were randomly allocated to rhIGF-1/rhIGFBP-3 (50µg/ml solution) or standard of care (SOC). Serial cranial ultrasounds were performed between birth and term-equivalent age. Presence of germinal matrix haemorrhage and intraventricular haemorrhage (GMH-IVH), periventricular haemorrhagic infarction (PHI), post-haemorrhagic ventricular dilatation (PHVD), and white matter injury (WMI) were scored by two independent masked readers.

Eligible infants had a gestational age (GA) at birth ranging from 23 weeks + 0 days to 27 weeks + 6 days. Exclusion criteria included detectable gross malformation, known or suspected chromosomal abnormality, clinically significant neurological disease, GMH-IVH grade II or III, or PHI (infants with grade I GMH-IVH were included).

Infants in the active treatment group received a standardized dosage of 250 µg/kg per day of rhIGF-1/rhIGFBP-3 via continuous intravenous infusion in addition to SOC from 24 hours of birth until a postmenstrual age (PMA) of 29 weeks + 6 days.

Infants in the control group received SOC based on their individual medical needs and according to local protocols.

A post-hoc analysis was conducted to further explore the phase II study findings relative to IVH.

**Table 1 is analysis of those infants that received treatment within the required therapeutic range**

| Treated IGF-1/IGFBP-3 are shaded | | | | | | |
|---|---|---|---|---|---|---|
| Control i.e. standard of care unshaded | | | | | | |
| **Gestational Age** | **n** | **No "bleed"** | **Grade1** | **Grade 2** | **Grade 3** | **PHI (Grade 4)** |
| **23** | **8** | **2** | **3** | **2** | **0** | **1** |
| | **3** | **1** | **0** | **2** | **0** | **0** |
| **24** | **14** | **9** | **0** | **1** | **1** | **3** |
| | **17** | **11** | **5** | **0** | **0** | **1** |
| **25** | **9** | **1** | **4** | **3** | **0** | **1** |
| | **13** | **8** | **1** | **2** | **0** | **2** |

| **Out of 23 treated infants:** | | **Out of 21 untreated infants:** | |
|---|---|---|---|
| | • PHI = **12.5%** | | • **16%** had PHI |
| | • Grade 3 IVH **=0%** | | • **3%** had grade 3 |
| | • Grade 2 IVH = **12%** | | • **19.4%** had grade 2 |
| | • Grade 1 IVH = **18%** | | • **21%** had grade 1 |
| | • No IVH = **60%** | | • **38.7%** No IVH |
| No IVH and grade 1 for treated = **78% Grade 3 and PHI =12.5%** | | No IVH and grade 1 for untreated **=59.7% Grade 3 and PHI =19%** | |

**Table 2**

| Treated IGF-1/IGFBP-3 are shaded | | | | | | |
|---|---|---|---|---|---|---|
| Control i.e. standard of care unshaded | | | | | | |
| **Gestational Age** | **n** | **No "bleed"** | **Grade 1** | **Grade 2** | **Grade 3** | **Grade 4** |
| **26** | **15** | **11** | **0** | **1** | **1** | **2** |
| | **16** | **14** | **0** | **1** | **0** | **1** |

| **Out of 16 treated infants:** | | **Out of 15 untreated infants:** | |
|---|---|---|---|
| | • PHI = **6.25%** | | • **13.3%** had PHI |
| | • Grade 3 IVH **=0%** | | • **6.6%** had grade 3 |
| | • Grade 2 IVH **= 6.25%** | | • **6.6%%** had grade 2 |
| | • Grade 1 IVH **= 0%** | | • **0%** had grade 1 |
| | • No IVH = **87.5%** | | • **73.3%** No IVH |
| No IVH and grade 1 for treated = **87.5% Grade 3 and PHI =6.25%** | | No IVH and grade 1 for untreated **=73.3% Grade 3 and PHI =19.9%** | |

This data shows that treatment of infants with low gestational age 23 to 25 weeks are more protected from incidences of IVH than those infants who receive only the standard of care 60% vs 38.7% respectivley, and incidence of grade 2, 3 and 4 IVH is also reduced in the patient population.

Infants born at 26 weeks were protected against grade 3 and grade 4 incidences of IVH by treatment with IGF-1/IGFBP-3 (6.25% vs 19.9%).
GMH-IVH graded according to Volpe method

### Severity Description

- **Grade I**: GMH with no or minimal IVH (<10% of ventricular area on parasagittal view)
- **Grade II**: IVH in 10-50% of ventricular area on parasagittal view
- **Grade III**: IVH in >50% of ventricular area on parasagittal view; usually distends lateral ventricle
- **Grade IV**: (IVH and PHI) IVH compounded by haemorrhagic venous infarction in the periventricular white matter

### Example 2 Summary

### Pre-term Rabbit Pups treatment with IGF-1/IGFBP-3

Preterm rabbit pups were delivered by caesarean section on E29 (term 32 days). IVH was induced by an i.p. administration of a 50% glycerol solution at 6 to 24-hours post-partum and verified by high-frequency ultrasound (HFU) at 24 hours. IVH initiating hyperosmolarity was induced by i.p. administration of a 50% glycerol at 6-hours post-partum. IGF-1/IGFBP-3 (8 mg/kg) (n=38) or vehicle (n=39) were s.c. administered from 3 hours of age and every 12 hours onwards, see Figure 4B. The extent of any haemorrhage was scored *in vivo* by HFU at 48 hours and assessed post-mortem by histopathological examinations.

The rate of induced IVH was highest at 6 hours post-delivery (66%) with a decreasing incidence at 12-24 hours, which is why it was selected, see Table 4.

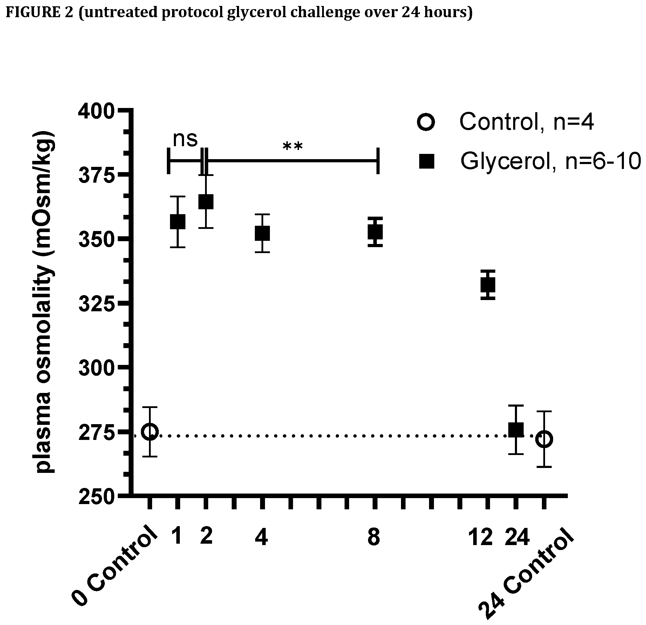

Plasma osmolarity increase without treatment is shown in Figure 2. Plasma osmolarity increased from 275 (±9.6) to 364 (±10.2) mOsm/kg, peaked at 2 hours and decreased to baseline (dotted line) between 12-24 hours.

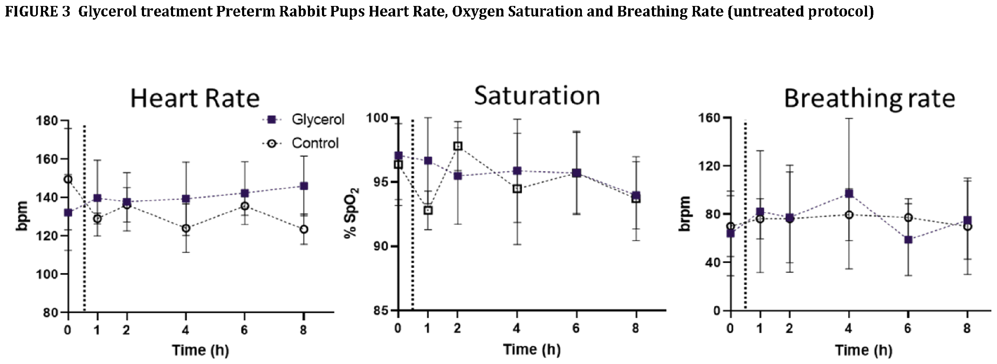

Heart rate, oxygen saturation and breathing rate data without treatment is shown in Figure 3.

Nevertheless, it may take up to 48 hours after initiation for the treatment to start being effective, whereas this model only provides treatment 3 hours prior to glycerol insult. Nevertheless, IGF-1/IGFBP-3 treatment inferred a huge benefit in preventing mortality. See Figure 5.

The data on IVH shows a numerical trend that treatment may provide protection against IVH about 48 hours post-glycerol-administration is initiated, see Table 3.

**Table 3**

| **Treatment group/ Number of animals administered with glycerol (n=)** | | **IVH incidence after 24h (n/n and %)** | | **IVH incidence after 48h (n/n and %)** | |
|---|---|---|---|---|---|
| | **rhIGF-1/rhIGFBP-3,** n=38 | 8/38 | 21% | 8/38 | 21% |
| | **Vehicle,** n=39 | 10/39 | 25.6% | 11/39 | 28.2% |

### Materials and methods

### Animals

Animal studies were conducted in accordance with and approved by the Swedish Animal Ethics Committee in Lund, Sweden. Reporting follows the ARRIVE guidelines. A well-established rabbit model for preterm birth, using a half-breed between New Zealand White and Lop rabbits (Löberöd, Sweden), with glycerol-induced intraventricular hemorrhage (IVH) were used as previously described.

Briefly, the experiments were performed on a total of 139 rabbit pups from 20 litters delivered via caesarean section (c.s.) after the does were anesthetized with i.v. propofol (5 mg/kg, Primen Pharmaceuticals Oy, Helsinki, Finland) on day 29 (full term = 31-32 days). After delivery, the pups were handled, fed and nursed for by highly experienced animal laboratory staff. The pups were dried and placed in an infant incubator set to a temperature of 30°C and 60% humidity.

### Experimental Setup:

Following preterm birth, animals were marked and randomized to experimental groups, treatment regimens, and termination time-points with equal distribution based on their bodyweights and litter. The experimental setup of experiments (A) and (B) are shown in detail in Figure 4.

### A. Investigation on the IVH-induction window

In Experiment A, the animals were subcutaneously (s.c.) injected after birth with a single bolus (100 uL) of sterile isotonic saline (0.9% NaCl, B Braun, Melsungen, Germany). At approximately 5 hours after birth, animals were monitored with high-frequency ultrasound (HFU, Vevo 2100, VisualSonics Inc., Toronto, ON, Canada) with a MS-550D 40 MHz transducer to scan for any spontaneous cranial bleedings as described previously. All non-bleeding animals received a single bolus of 50% (v/v) sterile glycerol solution (6.5 g/kg, Teknova, Hollister, US) given as an intraperitoneal injection (i.p.) at 6, 12, 18 or 24 hours after birth depending on the allocated experimental group. Two consecutive ultrasound scans were performed at 12 and 24 hours post-glycerol administration. Animals were terminated 24 hours post-glycerol administration, corresponding to a postnatal age of 30 (6 hour glycerol administration group), 36 (12 hour glycerol administration group), 42 (18 hour glycerol administration group) or 48 (24 hour glycerol administration group) hours, respectively.

### B. Preventive effect of rhIGF-1/rhIGFBP-3 on glycerol-induced IVH

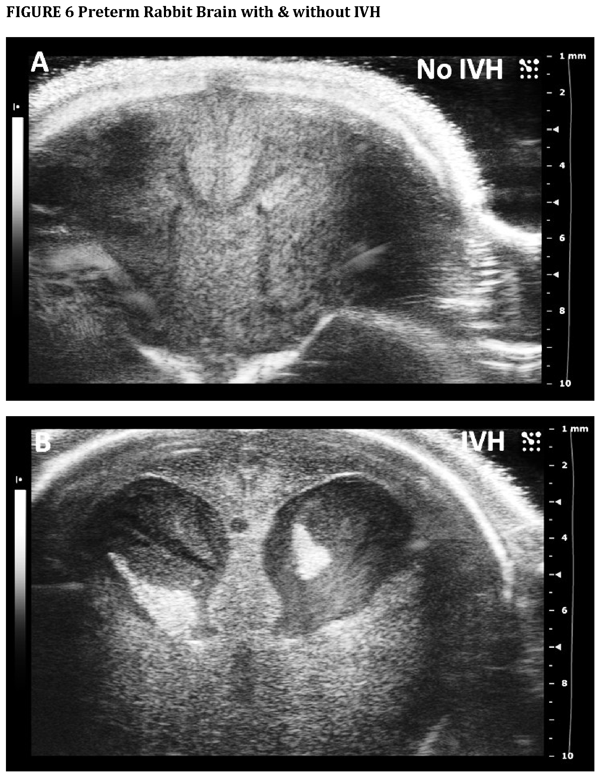

In Experiment B, the animals were s.c. administered with recombinant human (rh) IGF-1/rhIGFBP-3 (supplied, formulated and prepared in vehicle solution as described by Takeda Pharmaceutical Company Ltd, Boston, MA, USA) at 8 mg/kg or saline vehicle (0.9% NaCl, B Braun) at approximately 3 hours of age, and thereafter every 12 hours at an additional 4 consecutive time-points (for a total of 5 administrations). A single i.p. bolus of 50% glycerol solution (6.5 g/kg) was administered at 6 hours of age, 3 hours after the first rhIGF-1/rhIGFBP-3 administration. HFU scans were performed as described above every 24 hours post-glycerol administration to monitor the severity of the IVH. Pups with severe IVH on the ultrasound were assigned to the IVH group (Figure 6B), and those without detectable IVH at all time-points were used as controls (Figure 6A). Reproducibility and accuracy of ventricular measurements in this animal model using HFU have been described previously. The determination of the extent of bleeding were scored according to a simplified version of the standardized scale from Volpe. Figure 6 displays a representative ultrasound image of a rabbit pup without and with an IVH. All examinations were performed blinded to the operator. Animals were terminated 48 hours post-glycerol administration at a postnatal age of 54 hours. Animals that were euthanized due to poor physical health status, or died prior to scheduled termination, were subject to post-mortem ultrasound examinations.

### Tissue collection and processing

Animals were euthanized by decapitation at respective end-point (as determined by the experimental groups). Blood was collected from gravitation in Li-Heparin and Serum tubes (Microvette, Sarstedt. Germany), centrifuged and subsequently plasma and serum were immediately frozen and stored at -80°C. An ear biopsy (for sex determinations) was collected, snap frozen and stored at -80°C.

Following termination, brains were excised from the skull and immersion fixated for 24 hours in freshly prepared 4% paraformaldehyde solution (PFA, in 0.1 M phosphate buffer, pH 7.4). A change to fresh PFA was done after 3-6 hours and brains were immersed for a total of 24 hours, at 4°C. Brains were then cryo-protected by sequential immersion in 15% sucrose (diluted in phosphate buffer saline, PBS, 0.1 M, pH 7.4) for 6 hours and in 25% sucrose (diluted in PBS) for another 6 hours. Brains were mounted in TissueTec (Sakura Finetek, Torrance, CA, USA) and frozen (at around -60°C) in cryomolds, on dry ice in isopentane (2-methylbutane, Sigma-Aldrich, St. Louis, MO, USA). Sections (12 µm) were cut on a cryotome (Microm, HM 500OM, Microm Laborgeraete GmbH, Walldorf, Germany). Sections, were collected on SuperFrost plus slides (Menzel, Braunschweig, Germany). The brains were cryo-sectioned by coronal dissection in three blocks (forebrain, midbrain and hindbrain) and stored at -20°C.

### Hematoxylin-Eosin

To define the brain neuroanatomy and thereby enable macroscopic evaluation, sections were stained with hematoxylin-eosin (HE). The HE staining procedure was performed as follows: Sections were air-dried, at room temperature (RT) or 37°C, for 20-30 minutes, rinsed in PBS, 2x5 minutes, followed by a rinse in dH₂O for 1 minute. Sections were immersed in Mayers Hematoxylin (Histolab, Gothenburg, Sweden), for 2 minutes, followed by three fast 1 minute rinses in dH₂O. Sections were then immersed in sodium bicarbonate (0.1%), for 1 minute followed by immersion in dH₂O for 2x1 minute. Sections were immersed in 70% ethanol for 2 minutes, in Eosin (Histolab, 0.2% diluted in 70% ethanol acidified with glacial acetic acid) for 3 minutes. Sections were dehydrated in alcohol (96% x2 and 100% x2 for 3 minutes in each solution), and in xylene (100% for >10 minutes). Sections were mounted in Pertex (Histolab) and cover slipped.

### Peroxidase histochemistry

To detect peroxidase (P0) activity, and increase thereof by Hb, and to be able to determine its distribution in the brains of all animal groups we performed an adapted protocol of the enhanced peroxidase reaction of cryosections. Briefly, sections were air-dried, at RT or 37°C, for 20-30 minutes, rinsed in PBS 2x10 minutes. The peroxidase reaction was performed in a solution containing 3,3'-diaminobenzidine (DAB, 0.5 mg/ml diluted in PBS, Sigma-Adrich) containing 0.015% H₂O₂ (Merck, USA), for 10 minutes at RT. Sections were then rinsed in PBS 3x5 min, and in H₂O for 1 minute. Counterstaining was performed with hematoxylin (HTX, Mayers, Histolab), via immersion of sections in HTX for 2 minutes, and rinses in dH₂O followed for 3x1 minute. Sections were then dehydrated in alcohol (70% for 1 minute, 96% for 2x5 minutes and 100% for 2x7 minutes), and in xylene (100% for 2x5 min). Sections were mounted in Pertex (Histolab) and cover slipped.

### Histology evaluations

Histological evaluations of the degree of IVH were undertaken to compare and validate the HFU examination. The evaluations were made in three steps. First starting with an overall image analysis with macroscopic morphological assessment for the locality of any bleeding. Thereafter the whole brain was visually evaluated in the extent of any arachnoidal, parenchymal, ventricular, cerebellar or brainstem located bleeding. Subsequently evaluation of the degree of ventricular dilatation, findings of any tissue atrophy or necrosis and an overall scoring of bleeding in the three sectioned blocks. In the next step the different sections were stained with Hematoxylin/Eosin for determination of hemoglobin (Hb) leakage from erythrocytes and free Hb content, thereafter immunohistochemical labelling of PO for quantification of Hb in adjacent sections. The image analysis and scoring were performed blinded for the operator.

### Serum IGF-1 levels

The serum concentration of IGF-1 was determined using a human IGF-1 ELISA kit (Mediagnost, Reutlingen, Germany). The analysis was performed according to the manufacturer's instructions which states that the assay is applicable for rabbit serum samples.

### Sex determinations

Determinations of rabbit sex was done by confirming the presence of the sex determining region Y gene (gene ID: 100328958) in the rabbit genome using PCR and gel electrophoresis visualization as described before (1). Briefly, DNA was extracted using DNeasy Blood and Tissue Kit (Qiagen, Hilden, Germany) according to the manufacturer's instructions. One (1) ul of DNA was used in respective PCR reaction (30 cycles at 57°C) with the following primers: Sense: TGCAATACAGGAGGAACACG, Antisense: AGCAAACTGTCGCTCTTCTG. Presence of a band at approx. 299 bp was determined as male, and correspondingly no visual band was determined as female.

### Statistical power determination and data analysis

Based on the prevalence of IVH in Experiment A, a power estimation was made to calculate the group size for Experiment B. The aim was to obtain a treatment effect of 50%, and the calculations was based on numbers reported in randomized control clinical trial reported by Ley et al. (6). Calculations were made with power of (1-β = 0.80) and Type I error of (α = 0.05) generating the number of subjects and group size (see sample size in Results).

Statistical significance was calculated with a One-way ANOVA corrected for multiple comparisons (Bonferroni) or mixed effects analysis with a Two-way ANOVA using Dunnett's multiple comparisons test. Comparison of treatment group versus control group was evaluated using Student's t-test. Categorical outcome analysis was done on contingency data with Fischer's exact test with p-value and the difference between proportions as attributable risk in percent (%). When possible, animals that died or were euthanized prior to scheduled termination were included in the statistical analysis. P-values <0.05 were considered significant. Statistical analyses were performed using GraphPad Prism (GraphPad Prism 9; GraphPad, San Diego, US).

### Results

### Rate of IVH induction

In Experiment A, the incidence of developing an IVH in preterm rabbit pups after i.p. administration of a 50% glycerol solution was highest when administered at 6 hours after birth reaching an incidence of 66% (n=4 out of 6, see Table 4). At later time-points of induction (18 and 24 hours) the incidence decreased linearly (33%, n=2 out of 6; and 14%, n=1 out of 7, see Table 4). No animals in the group receiving glycerol at 12 hours (0%, n=0 out of 5, see Table 4) of age develop IVH in this experiment. Based on these results, a glycerol administration time-point of 6 hours post-birth was selected as the time-point for induction of IVH in Experiment B.

**Table 4**

| **Administration group/ Glycerol administered (n)** | **IVH incidence after 24h (n/n and %)** | |
|---|---|---|
| **6 hours** (6) | 4/6 | **66%** |
| **12 hours** (5) | 0/5 | **0%** |
| **18 hours** (6) | 2/6 | **33%** |
| **24 hours** (7) | 1/7 | **14%** |

The incidence of IVH (%) in Experiment A (n=5-7). Animals were i.p. administered a 50% glycerol bolus (6.5 g/kg) at 6, 12, 18 or 24 hours after birth. The extent of bleeding were assessed by high-frequency ultrasound 24 hours after glycerol admin. Cranial bleeding were scored as severe IVH or no IVH. i.p., intraperitoneal; IVH, intraventricular haemorrhage.

### Incidence of IVH and sex distribution.

In Experiment B, a numerical trend towards reduced occurrence of severe IVH could be observed in the rhIGF-1/rhIGFBP-3 group as compared to the Vehicle control group at 48 hours (21% vs. 28.2%, p-value 0.598). Of notice, the difference in rate of severe IVH was observed to increase between the rhIGF-1/IGFBP-3 and Vehicle group at 48 vs. 24 hours (7.2% vs. 4.6% respectively) (Table 3). Distribution of sex was found to be evenly distributed in the rhIGF-1/IGFBP-3 group, whereas there was a slightly uneven distribution, with a reduced number of males, in the Vehicle group (Table 5).

**Table 5**

| **Treatment No. of animals administered glycerol (n=)** | **IVH incidence after 24h (n/n and %)** | | **Distributi on, M/F (n)** | **IVH incidence after 48h (n/n and %)** | | **Distributio n IVH after 24h, M/F (n)** | **Distributio n IVH after 48h, M/F (n)** |
|---|---|---|---|---|---|---|---|
| **rhIGF-1/ rhIGFBP-3,** n=38 | **8/38** | **21%** | **17/21** | **8/38** | **21%** | **5/3** | **5/3** |
| **Vehicle,** n=39 | **10/39** | **25.6%** | **17/22** | **11/39** | **28.2%** | **2/8** | **3/8** |

Group size, incidence of IVH (%) and sex distribution in Experiment B, at 24 and 48 hours after glycerol administration in animals receiving either rhIGF-1/rhIGFBP-3 at 8 mg/kg (n=38) or Vehicle (n=39). p-values from Fischer's exact test. rhIGF-1, recombinant human (rh) insulin-like growth factor-1/rh insulin-like growth factor binding protein-3; IVH, intraventricular haemorrhage.

### Histological analysis confirms ultrasound evaluations

To validate the ultrasound examinations in the detection of IVH, or presence of additional bleedings within the parenchyma, brain tissue was evaluated by histological analysis. Macroscopic histomorphological evaluation displayed no bleedings in animals scored as no IVH in the ultrasound examination, as exemplified in Figure 6A. Furthermore, all animals scored as IVH in the ultrasound was confirmed as bleeding (as exemplified in Figure 6B). PO staining further confirmed the results of ultrasound and histomorphological examinations.

### rhIGF-1/IGFBP-3 increase survival

The survival at 48 hours following IVH was observed to be approximately 63.6% (n=7 out of 11) in the vehicle administered group, and 87.5% (n=7 out of 8) in the rhIGF-1/rhIGFBP-3 administered group (Figure 5, Table 6). In animals with no IVH, survival of vehicle administered animals was 93.1% (n=27 out of 29) compared to 100% (30 out of 30) in the rhIGF-1/rhIGFBP-3 administered animals (Figure 5, Table 6). The overall survival, *i.e.* regardless of developing an IVH or not, the survival in the vehicle administered animals was 87.2% (n=34 out of 39) as compared to 97.4% (37 out of 38) in the rhIGF-1/rhIGFBP-3 administered animals (Figure 5, Table 6).

**Table 6**

| **Treatment group (n=)** | | **Mortality at ≤54H in IVH animals (n/n and %)** | | **Mortality at ≤54H in no IVH animals (n/n and %)** | | **Overall mortality (IVH and no IVH) at ≤54H(n/n and %)** | |
|---|---|---|---|---|---|---|---|
| | **rhIGF-1/rhIGFBP-3,** n=38 | 1/8 | 12.5% | 0/30 | 0% | 1/38 | 2.6 |
| | **Vehicle,** n=39 | 3/11 | 27.3% | 2/28 | 7.1% | 5/39 | 12.8 |

### Serum levels of IGF-1

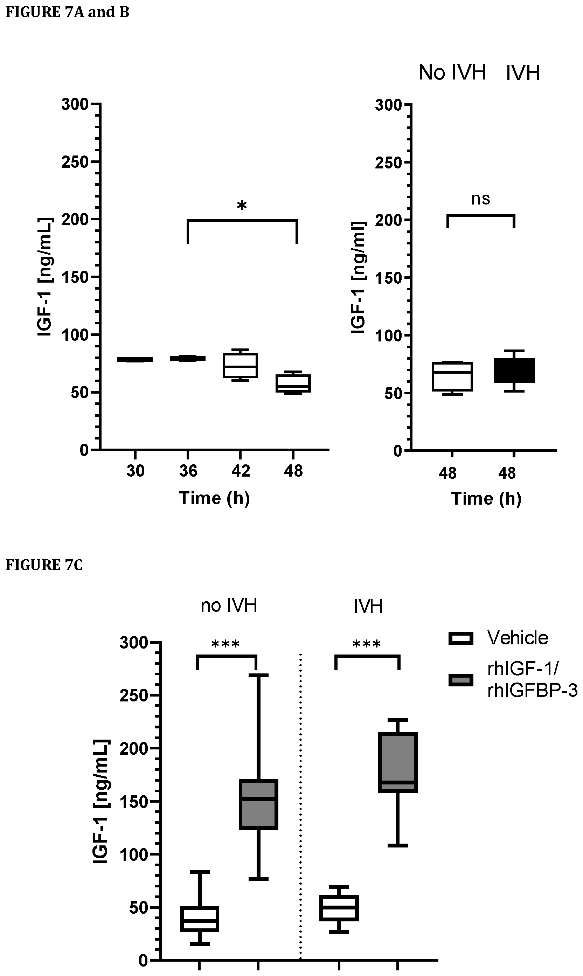

Circulating levels of endogenous IGF-1 in Experiment A display a significant decline in concentration after birth in the preterm rabbit pup (Figure 7A) from 78 ng/ml to 57 ng/ml (p=0.020) at 30 and 48 hours of life respectively. IGF-1 serum levels in animals that developed an IVH (70 ±12) were not significantly different from non-IVH animals (63 ±13, p=0.539, Figure 7B). These results correspond well with previous experiments where serum levels in rabbit pups of equivalent age range from 69 ng/ml to 45 mg/ml at 24 to 48 hours after birth respectively.

In Experiment B, serum levels of IGF-1 increased significantly following rhIGF-1/rhIGFBP-3 administration in both no IVH group animals (149±42; n=29) as well as IVH animals (179±41; n=7) as compared to vehicle administered animals (no IVH, 39±16, n=27; IVH, 48±15, n=7, p<0.001)(Figure 7C). Serum levels of IGF-1 were not statistically different comparing animals that had developed an IVH vs. no IVH animals (p= 0.18 and 0.30) following administration of rhIGF-1/rhIGFBP-3.

Endogenous serum levels of IGF-1 in preterm rabbit pups **(A)** from Experiment A, after administration of 50% glycerol. Blood were collected at decapitation at 30, 36, 42 or 48 hours after C-section. n= 2-5. Boxplot with min and max values. *p< 0.05.

Serum levels of IGF-1 **(B)** in preterm rabbit pups with IVH (n=9) or no IVH (n=4) at 30-48 hrs after C-section. Boxplot with min and max values. **(C)** Serum levels at 54 hours after birth of IGF-1 after rhIGF-1/rhIGFBP-3 (8 mg/kg) or Vehicle administration in Experiment B. Boxplot with min and max values. Unpaired t-test *** p<0,001. No significant difference comparing both administration groups (p=0.30 and 0.18; rhIGF-1/rhIGFBP-3 and Vehicle groups respectively). C-section, Caesarean section; IVH, intraventricular haemorrhage; IGF-1, Insulin-like growth factor-1. rhIGF-1/rhIGFBP-3, recombinant human (rh) insulin-like growth factor-1/rh insulin-like growth factor binding protein-3.

### EXAMPLE 3

Preterm rabbit pups (E29) were subjected to subcutaneous administration of labelled (FITC, biotin or Alexa Fluor-647) or unlabelled IGF-1/IGFBP-3 (8 mg/kg) and followed for 5 and 24 hours. Brains were perfused and investigated for presence of IGF-1 and interaction with the IGF-1R using confocal-, electron-, and light sheet microscopy. In addition, the CP was collected and analysed for IGF-1R activation using western blot

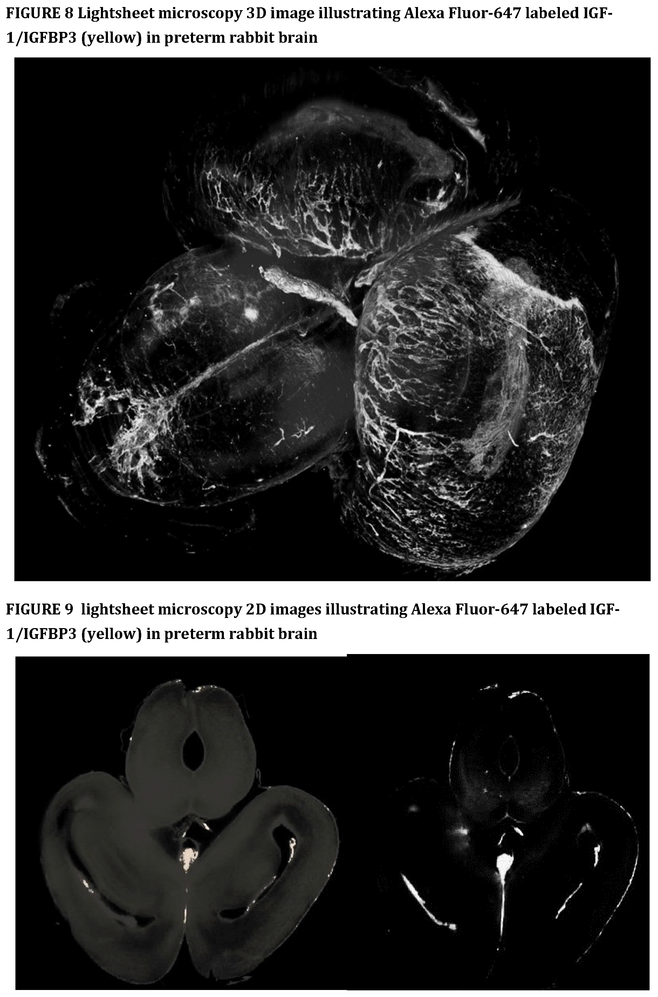

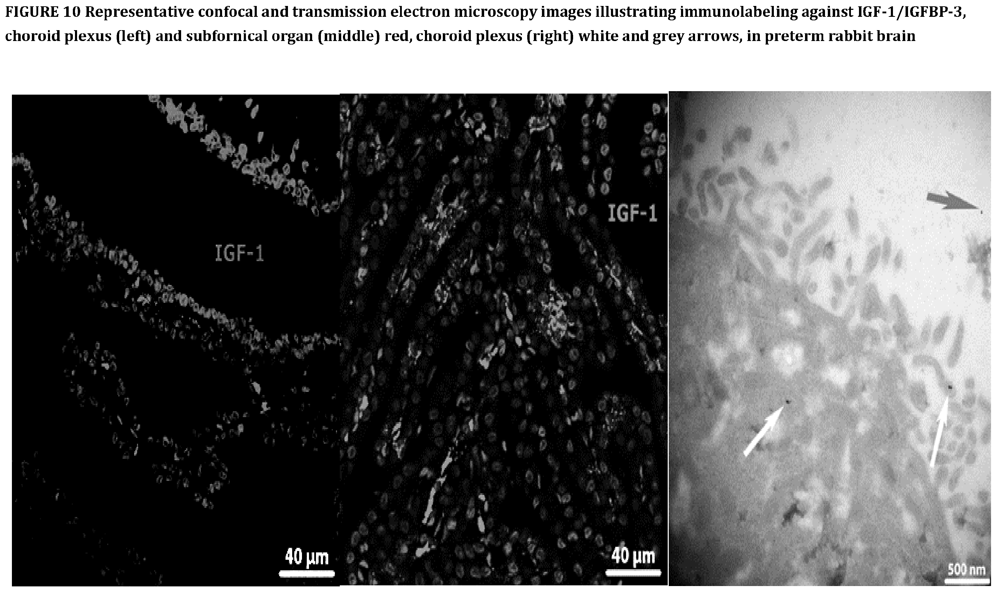

IGF-1/IGFBP-3, conjugated with Alexa Fluor-647 or biotin, was detected in the CP, the subfornical organ and the subarachnoid space five hours post-administration (Figures 8, 9 and 10). Immunolabeling for FITC-conjugated IGF-1 in electron microscopy revealed translocation of IGF-1/IGFBP-3 through the CP (Figure 10 light areas).

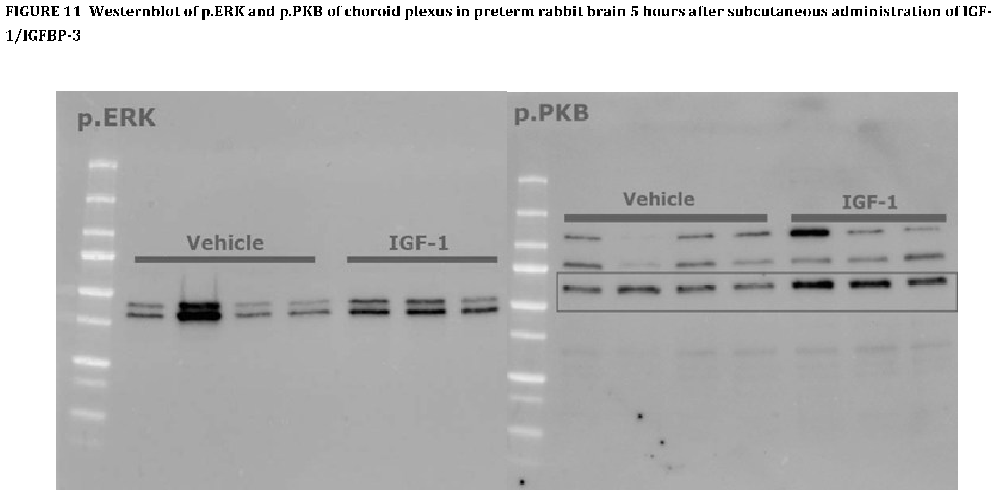

Western blot analysis displayed an increased activation, i.e. phosphorylation, of the IGF-1R downstream pathways MAP kinase (as shown by p.ERK) and PI 3-kinase (as shown by p.PKB) following exposure to IGF-1/IGFBP-3 (Figure 11).

Blood-borne exposure of IGF-1/IGFBP-3 binds to and translocates across the CP as well as activates its IGF-1 receptor i the preterm rabbit brain.

This indicates potential impact of systemically administered IGF-1/IGFBP-3 on the development of the immature brain.

### EXAMPLE 4 Choroid plexus extracellular vesicle transport of blood borne insulin-like growth factor 1 to the hippocampus of the immature brain

### Materials & Methods

### Preterm rabbit pups

The animal protocols were approved by the Swedish Animal Ethics Committee in Lund (Dnr: 5.8.18-06020/2019). We used the well-established preterm rabbit pup model in accordance with the previous description (42). The New Zealand White was used (Egle Kergiene, Lundsbrunn, Sweden). Briefly, the experiments were performed on a total of 9 rabbit pups from 7 litters (4 females and 4 males) delivered via cesarean section (c.s.) after the does were anesthetized with intravenous Propofol-Lipuro (20 mg/ml i.v., B. Braun Melsungen AG, Melsungen, Germany) on day 29 (term 31-32 days). After delivery, the pups were handled and nursed by the animal laboratory staff. The pups were dried and placed in an infant incubator set to 30°C and 60% humidity. At approximately. 1-2 hours of age, the pups were weighed, marked, and hand-fed with bovine colostrum (100 ml/kg/day, Biodane Pharma, Gesten, Denmark) using a 4 French feeding tube (Vygon, Ecouven, France). At 12 hours of age, the pups received a mixture (1:1, v/v) of bovine colostrum and Fox Valley 30/50 (Melk voor Dieren, Rotterdam, the Netherlands). From 24 hours and onwards, pups received only Fox Valley 30/50. The administered amount of Fox Valley 30/50 was increased every 24 hours by 10 ml/kg/day. Feeding at 12, 36 and 60 hours of age were reduced to half of the previous corresponding dose, i.e., 50 ml/kg at 12 hours of age (corresponding to half of 100 ml/kg given at birth) etc. Pups were gently cleaned once or twice a day, as required to maintain hygiene.

### Preterm piglets

All animal procedures were performed in accordance with the Danish National Committee on Animal Experimentation (license no. 2014-15-0201-00418). Preterm piglets were delivered by cesarian section at 106 day of gestation (full term = 117 days), housed in individual incubators and reared to either 5 (P5) or 9 days (P9) as previously described in L. I. Christiansen et al., Insulin-Like Growth Factor-1 Supplementation Promotes Brain Maturation in Preterm Pigs. eNeuro 10 (2023).. Pigs were treated with either vehicle (control group) or recombinant human (rh)IGF-1/rhIGFBP-3 complex (2.25 mg/kg/d; mecasermin rinfabate, Takeda) as continuous systemic infusion either via an intra-arterial catheter until P5 and subcutaneously three times per day until P9. Detailed feeding regiment and physiological, behavior and clinical responses to IGF-1 treatment, including brain, gut, metabolic, and immune effects are reported in separate articles (In Christiansen *et al.* above & K. Holgersen et al., Clinical outcome and gut development after insulin-like growth factor-1 supplementation to preterm pigs. Front Pediatr 10, 868911 (2022). P9 pigs (used in this study) were anesthetized and then sacrificed with intracardiac injection of sodium pentobarbital followed by immediate collection of CSF by suboccipital puncture. The CSF was centrifuged at 2500 x g at 4°C for 10 min, visually inspected for stick bleedings (to exclude blood contamination) and stored at -80°C.

### Primary ChPE cell cultures

The animal protocols were approved by the Swedish Animal Ethics Committee in Lund, Sweden (Dnr. 5.8.18-12,930/2019). Primary murine ChPE cell culture studies were performed as previously described (17). In brief, brains from 3-8-day old pups (C57Bl/6Ncrl, Scanbur, Karlslunde, Denmark) were isolated. ChP from lateral and fourth ventricle were isolated under a dissection microscope

(Nikon SMZ800N Stereomicroscope, Tokyo, Minato, Japan). The cells were further dissociated by the enzymatic reaction of 2 mg/ml pronase (isolated from Streptomyces griseus, Merck, Burlington, MA, USA). The reaction was terminated by the addition of excess complete DMEM-F12 cell culture medium (Gibco, Waltham, MA, USA) containing 10% fetal bovine serum (FBS, Gibco) and 1 % Antibiotic-Antimycotic (Gibco). The cells were then centrifuged 1000 xg for 2 minutes, resuspended in DMEM-F12 culture medium and plated (105 cells/well) on a 12-well transwell system (CLS3460-48EA, Sigma). Cells were incubated at 37°C in 5% CO2 for 8-9 days. To eliminate fibroblast contamination, cell culture media were changed after 48 hours to complete DMEM-F12 culture media containing cytosine arabinoside (Ara-C, Merck). The complete DMEM-F12 culture medium was thereafter changed every 48 hours. Functional characteristics of the ChPE cells were ascertained by positive labeling for TTR and ZO-1, absent labeling for fibroblast markers (S100A4 and Hsp47) and an increasing TEER during culture (described in more details in "Trans epithelial electric resistance (TEER)).

### Transepithelial electric resistance (TEER)

TEER analysis was performed to ensure that ChPE cells retained/established blood barrier function and was conducted using the EVOM2 (World Precision Instruments, Sarasota, FL, USA). The probes were sterilized by incubating for 15 seconds to 1 minute in 70% ethanol and washed with phosphate buffered saline (PBS, pH 7.4). Identical sterilization steps were performed between every TEER measurement. TEER measurement of wells containing only complete DMEM-F12 cell culture medium was used as blanks and the obtained reading was subtracted from all measurements. The filter membrane diameter was multiplied to all blank subtracted measurement and the electric resistance was expressed as Ω.cm2.

### IGF-1 exposure of ChPE cell cultures

After 8-10 days of culturing of the ChPE cells, the cells reached electrical resistance readings of 80 - 110 Ω.cm2 and was considered ready for experimental exposure (14). The cell culture media was changed to complete DMEM-F12 containing 10% Exosome-Depleted FBS (Gibco) and the culture was maintained for 24 hours. Thereafter, 4 hours before IGF-1 exposure, the cell culture media was changed to DMEM-F12 containing 2% Exosome-Depleted FBS. 40, 100 or 250 ng/ml human IGF-1 (R&D systems, 291-G1, Minneapolis, MN, USA) was added to the basolateral area (corresponding to the circulatory/blood compartment), and the ChPE cell culture was exposed for 24 hours. Subsequently, the apical supernatant (corresponding to the CSF compartment) as well as the basolateral supernatant was collected and stored at -80°C until subsequent EV preparation and analysis (see "EV preparation" for further details).

### Diffusion assay

ChPE cell cultures were prepared as described in "Primary ChPE cell cultures". Next, transwell were transferred to a new multi-well plate and 800 µl phenol red-free MEM (Gibco) was added to the basolateral compartment. The cell culture medium of the apical compartment (MEM) was replaced with 250 µl of 1 mg/ml 20 kDa FITC-Dextran solution (Merck). The ChPE cells were incubated, protected from light, at room temperature for 20 minutes. Subsequently, the basolateral media was collected and 100 µl aliquots transferred to a 96-well microtiter plate in triplicates and the plate was read at 490 (excitation) and 520 nm (emission) using a VICTOR3 plate reader (Perkin Elmer, Waltham, MA, USA).

### EV preparation

To prepare EVs from ChPE cell culture supernatant, the miRCURY exosome kit (Qiagen, Hilden, North Rhine-Westphalia, Germany) was used according to the manufacturer's instructions. In brief, samples were centrifuged for 8 minutes at 3000 xg to remove debris. Precipitation media (0.4x) was added to the supernatant and incubated over-night at 4°C. The precipitation solution was centrifuged at 10 000 xg, 20°C and the supernatant was carefully aspirated and stored at - 80°C. The EV pellet was resuspended in 40-100 µl of provided suspension media and stored at - 80°C or further prepared for NTA. For subsequent MS analysis, the pellets were directly stored at -80°C.

### EV number and size measurements

To determine EV size and concentration, NTA was used. In brief, EVs were prepared as described above, diluted 1/10 in PBS and injected into NanoSight (LM14C, Malvern Panalytical, Malvern, U.K.). All samples were recorded for 90 seconds in triplicates with camera level 15 and detection threshold 5. Absolute numbers were calculated with the acquisition software (NTA version 3.3, Malvern Panalytical).

### Immunofluorescence microscopy and image analysis

Primary ChPE cells were fixed in 4% buffered paraformaldehyde (PFA prepared in PBS, pH 7.4) for 15 minutes and washed three times with PBS. The cells were thereafter permeabilized with 0.1% TritonX-100 (0.1 % in PBS, Invitrogen, Waltham, MA, USA) for 10 minutes, subsequently blocked with 10% FBS for 1 hour, and incubated with primary and secondary antibodies (as described below) over-night at 4°C and 1 hour room temperature, respectively. The following primary antibodies were used: Hsp47 (1:200, Rabbit anti-Mouse, ab109117, Abcam, Cambridge, UK), S100A4 (1:250, Rabbit anti-Mouse; ab197896, Abcam), IGF-1 (1:100, rabbit-anti-Mouse, bs-0014R, Bioss, Woburn, MA, USA), IGF-1 (1:100, Goat anti-Mouse, AF791, R&D systems), IGF-1R (1:20, Goat anti-Mouse, AF-305-NA, R&D systems), ZO-1 (1:50, Rabbit anti-Mouse, 61-7300, Thermo Fisher Scientific, Waltham, MA, USA), Donkey anti-Goat 647 (1:500, A-21447, Thermo Fisher Scientific), TTR (1:50, Sheep anti-Mouse ab9015, Abcam), CD63 (1:100, Rabbit anti-Mouse, ab217345, Abcam). The following secondary antibodies were used with the corresponding primary antibody: Donkey-anti-Goat 488 (1:500, A-11015, Thermo Fisher Scientific), Goat anti-Rabbit 488 (1:500, ab150077, Abcam), Goat anti-Rabbit 568 (1:500, ab175471, Abcam) and Donkey anti-Sheep 488 (1:400, A-11015, Thermo Fisher Scientific). The membranes were counterstained with Hoechst (1:10 000 in DMSO, H1398, Invitrogen) for 5 minutes at room temperature and mounted using mounting media (Merck). To visualize the samples, a Nikon Confocal A1RHD confocal (Nikon, Minato, Tokyo Japan) microscope was used.

### Electron microscopy

ChPE derived EVs were prepared as described in "EV preparation". 30 µl of an EV preparation was fixed with 30 µl 4% PFA for 30 minutes at room temperature. A drop (approx. 5 µl) of fixed EV suspension was placed on Formvar-carbon coated electron microscopy grid to air dry for 20 minutes at room temperature. Next, to wash the samples, the grid was placed to float over a drop of 100 µl PBS, placed on clean parafilm surface. All subsequent steps were performed in the same manner.

The grids were blocked with 1% bovine serum albumin (BSA, diluted in PBS), incubated with primary antibody against IGF-1 (1:100, Rabbit anti-Mouse, bs-0014R, Bioss) and Flotillin 2 (5 ug/ml, Rabbit anti-Mouse, ab96507, Abcam) for 120 minutes at room temperature. Incubation with secondary antibody, Goat anti-rabbit IgG (H+L) 10 nm gold conjugate (17010-1, Ted Pella, Redding, CA, USA) was performed for 60 minutes at room temperature.

ChPE cells cultured as described in "Primary ChPE cell cultures" was prefixed with 4% PFA for 1 hour and subsequently rinsed several times with Sorensen phosphate buffer (0.1 M). Cells were thereafter dehydrated with acetone in distilled water in a 6-step series with increased acetone concentration per step (30 - 100%). Time per step were 5- 10 minutes. Cells were then impregnated over night with a 1/1 mixture if acetone and Epon (Agar Scientific Ltd, Stansted, Essex England). Next, the cells were embedded in Epon and then polymerized in Epon for 48 hours at 60 °C and ultra sectioned to 60nm thick sections. Blocking was subsequently conducted for 1 hour using 1% BSA in distilled water and subsequently cells were incubated with primary antibodies against IGF-1 (1:100, Goat anti-Mouse, AF791, R&D systems) (1:100, Goat anti-Human, AF291, R&D systems) and Flotillin 2 (10 ug/ml, Rabbit anti-Mouse, ab96507, Abcam) overnight at 4 °C. Incubation with secondary antibody, Rabbit anti-goat IgG (H+L) 10 nm gold conjugate (1/20,17410-1, Ted Pella, Redding, CA, USA) and Goat anti-rabbit IgG (H+L) 10 nm gold conjugate (1/20, 17010-1, Ted Pella, Redding, CA, USA) was performed for 60 minutes at room temperature. The sections were stained with uranyl acetate (4%, Agar scientific) for 20 minutes at 38 °C. All samples including EVs and ChPE cells were examined in a FEI Technai Biotwin 120kv TEM operated at 100 kV accelerating voltage. Images were recorded with side-mounted Olympus Veleta camera with a resolution of 2048 × 2048 pixels (FEI, Hillsboro, OR, USA).

### Liquid chromatography-mass spectrometry

100 µL Ripa buffer (R0278, Sigma-Aldrich) was added to samples prepared as described in "EV preparation" and subsequently sonicated using a Bioruptor (Diagenode), 40 cycles (15 sec on, 15 sec off). Samples were reduced with dithiothreitol to a final concentration of 10 mM and heated at 56 °C for 30 min followed by alkylation with iodoacetamide to a final of 20 mM for 30 min at room temperature in dark. Samples were precipitated with ice cold ethanol (final concentration of ethanol 90%) overnight at -20 C followed by centrifugation at 14 000 x g for 10 min. The pellets were air dried and resuspended with 50 µL of 100 mM ammonium bicarbonate and sonicated using a Bioruptor (Diagenode), 40 cycles (15 sec on, 15 sec off). Protein concentration was measured at 280 nm using a NanoDrop (DeNovix DS-11, DeNovix Inc; Wilmington, DE, USA). Digestion was performed by adding trypsin in a ratio of 1:50 (Sequencing Grade Modified Trypsin, Part No. V511A, Promega) to the samples and incubated overnight at 37 °C. The digestion was stopped by 5 µL 10% trifluoroacetic acid. The samples were Speed Vac to dryness and resolved in 2% ACN, 0.1%. Peptides extracted were analyzed on an Exploris 480 mass spectrometer (Thermo Fischer Scientific) coupled with a Vanquish Neo UHPLC system (Thermo Fischer Scientific). Two-column setup was used on the HPLC system and peptides were loaded into an Acclaim PepMap 100 C18 precolumn (75 µm x 2 cm, Thermo Scientific, Waltham, MA) and then separated on an EASY spray column (75 µm x 25 cm, C18, 2 µm, 100 Å, ES902) with the flow rate of 300 nL/min. The column temperature was set 45 °C. Solvent A (0.1% FA in water) and solvent B (0.1% FA in 80% ACN) were used to create a 90 min nonlinear gradient from 5 to 25% of solvent B for 75 min and increased 32% for 9 min and the increased to 45% for 6 min to elute the peptides.

The samples were analyzed with a data-dependent acquisition (DDA) in positive mode. The full mass spectrometry spectra 1 (MS1) resolution were set to 120,000 at m/z 200 and the normalized AGC target was set to 300% with the maximum injection time of 45 ms. The full mass range was set 375-1500 m/z. Precursors were isolated with the isolation window of 1.3 m/z and fragmented by HCD with the normalized collision energy of 30. The full mass spectrometry spectra 2 (MS2) was detected in the Orbitrap with the resolution of 15,000. The normalized AGC target and the maximum injection time were set to 100% and custom, respectively. The intensity threshold for precursor selection was set to 1e4 and 40s dynamic exclusion was applied.

### Data analysis of mass spectrometry data

The raw DDA data were analyzed with Proteome Discoverer^{™} 2.5 Software (Thermo Scientific, Waltham, Massachusetts, USA), and the peptides were identified using SEQUEST HT against UniProtKB Mouse canonical database (UP000000589) and fasta files for the human IGF1 (P05019). The search was performed with the following parameters applied: carbamidomethylation. of cysteine as static modification: and N-terminal acetylation and methionine oxidation as dynamic modification. Precursor tolerance was set to 10 ppm and fragment tolerance was set to 0.02 ppm. Up to 2 missed cleavages were allowed and Percolator was used for peptide validation at a q-value of maximum 0.01. Extracted peptides were used to identify and quantify them by label-free relative quantification. The extracted chromatographic intensities were used to compare peptide abundance across samples.

Protein abundancies were submitted to the online tool Proteomill (https://proteomill.com) (44). Missing values were set to that each protein had a minimum of four 6 values in each group. Enrichment analysis was performed by searching the GO, David and Metascape databases for Reactome pathways and GO terms (24-26, 45).

### In vivo intraventricular brain injections

EVs prepared as described in "IGF-1 exposure of CPE cell cultures" and "EV preparation" were stained with PKH26 Red Fluorescent Cell Linker Mini Kit for General Cell Membrane Labeling (Merck, MINI26) as described by the manufacturer. Briefly, 400 µl of ChPE supernatant were mixed with PBS and PKH26 (1:200) in a total volume of 800 µl and subsequently let to incubate for 20 minutes at room temperature. This was followed by exosome isolation as described in "EV preparation" and pellet resuspension in 50 µl PBS. By guidance with a high-resolution ultrasoundRU (Vevo 2100, VisualSonics Inc., ON, Canada) with a MS-550D 40 MHz transducer, non-sedated preterm rabbit pups with the postnatal age of 24 hours, received an i.c.v. injection of 25 µl stained EVs using a BD Microfine + 0.3 ml (30 gauge). The preterm rabbit pups were followed for 4.5 hours and anaesthetized with an intramuscular (i.m.) injection of Ketaminol vet.

(50 mg/ml, Intervet AB, Stockholm, Sweden) and Rompun vet. (20 mg/ml, Bayer Animal Health, Leverkusen, Germany) in combination with isoflurane inhalation (Attane vet, 1000 mg/g, VM Pharma AB, Stockholm, Sweden). Following sedation, the pups were transcardially perfused with freshly prepared PBS (containing heparin 1000 IU/ml), followed by perfusion with freshly prepared 4% paraformaldehyde (PFA, VWR Chemicals, Leuven, Belgium, buffered with PBS, pH 7.4). After fixation, the brains were extracted from the skull and post-fixed by immersion in 4% PFA. A change to fresh PFA was performed after 6-8 hours and brains were then immersed in PFA for a total of 24 hours at 4°C and transferred to PBS for further processing as described in "Histological detection of administrated EVs in the brain of rabbit pups" below.

### Histological detection of administrated EVs in the brain of rabbit pups

Brains were excised and were immersed whole in PFA (4% in PBS, pH 7.4; PFA) for 16 hours at 4°C. Brains were subsequently disvided in two pieces, coronally in the midbrain (close to the location of the subfornical organ, that were incubated in PFA for 4 hours at room temperature. Next, brains were rinsed in PBS, pH 7.4, for 8 hours at 4°C, embedded in OCT cryomount (Histolab, Sweden) and frozen on dry ice in isopentane (approximately at - 60 °C). Sections were cut (10 µm thick) through the midbrain with a cryostat and sections holding the lateral and central ventricles with the choroid plexus and the hippocampus were collected on SuperFrost Plus slides (Thermo Scientific/Gerhard Menzel B.V. & Co., Braunschweig, Germany). Following rinses in PBS (2 x 5 min) and sections (3-6 per animal) were incubated in 4',6-diamidino-2-phenylindole (DAPI) for 15 minutes at room temperature and were subsequently mounted and cover slipped in Fluoroshield (Abcam) anti-fade mounting media. Analyses were performed with a confocal laser scanning microscope (Zeiss LSM 800, ZEIZZ, Oberkochen, Germany). Detection was performed of PKH26 (stained vesicles), set for excitation maxima at 551 nm and emission maxima at 567 nm. Sequential scanning was performed of PKH26 and DAPI for analyses of the distribution and location of PKH26 fluorescence, primarily in the ChP, ventricular brain ependyma and the parenchyma of the hippocampus. Both hemispheres were analyzed. Threshold detection level of PKH26 was set in brain sections from animals that received only the dye (no vesicles). Representative images were grabbed and converted to TIFF format for illustrations.

### In vitro EV uptake experiment with hippocampal neurons

Wistar rats were obtained from Charles River (Sulzfeld, Germany) and handled in accordance with the Danish Animal Welfare Act approved by the Department of Experimental Medicine at the University of Copenhagen (Copenhagen, Denmark).

Primary hippocampal neurons were isolated from embryonic day 19 Wistar rat embryos in accordance with (46). Dissected hippocampi were chopped in ice-cold Krebs-Ringer buffer (KRB, Invitrogen) and treated with 0.1% (w/v) trypsin for 6-7 minutes at 37°C, followed by incubation with 0.052% (v/w) trypsin inhibitor and 0.008% (v/w) DNase I diluted in KRB. Undissociated tissue was pelleted by centrifugation, and the neurons were resuspended in KRB containing 0.13 mM Ca2+ and 2.4 mM Mg2+ (Invitrogen). Cells were pelleted by centrifugation and resuspended in NeurobasalTM medium supplemented with 2% (v/v) B27, 100 U/ml penicillin and 100 µg/ml streptomycin (all from Invitrogen). Neurons were then plated at density 5 × 104 cells/cm2 in eight-well LabTek Permanox chamber slides (Nunc, Roskilde, Denmark) pre-coated with 20 µg/ml poly-L-lysine and cultured for 8-10 days with half medium exchange at the second and seventh days in vitro.

For the uptake experiment, ChPE derived EVs were stained with PKH67 Green Fluorescent Cell Linker Mini Kit for General Cell Membrane Labeling (Merck, MINI67) as described by the manufacturer. Briefly, 400 µl of ChPE supernatant were mixed with PBS and PKH67 (1:200) in a total volume of 800 µl and subsequently let to incubate for 20 minutes at room temperature. This was followed by exosome isolation as described in "EV preparation" and pellet resuspension in 300 µl PBS. Subsequently, neurons were stimulated for 2 hours with the PKH67-stained EVs. For each slide, a well with unstimulated cells was used as control and wells with only PKH67-stained supernatant with no cells were used as negative control.

Cells were then washed with PBS, fixed with 4% PFA for 20 minutes, blocked with 5% BSA and labeled with polyclonal Rabbit anti-rat growth-associated-protein-43 (GAP-43) antibody (1:1000; Millipore) over-night at 4°C followed by incubation with secondary Goat anti-Mouse Alexa Fluor 546-conjugated antibodies (1:1000; Invitrogen). For visualization of nuclear morphology, cells were counterstained with Hoechst 33258 (1:1000; Invitrogen) and slides were mounted with antifade mounting medium (Dako, Glostrup, Denmark). Images were recorded in systematic series of fields of view across the whole area of a well using a ZEIZZ confocal laser scanning microscope (ZEIZZ LSM800, ZEIZZ, Oberkochen, Germany).

For PKHK67 fluorescence analysis, slides were scanned manually due to the unavailability to scan the plastic membranes. Focus was set manually for each image required. To obtain digital images, a wide-field epi-fluorescence microscope (Olympus IX70, Tokyo, Japan) equipped with a digital detector (DP80, Olympus) were used under fixed lighting conditions and regions of interest (ROI) were manually annotated. Images were exported as TIFF and imported to Fiji. Quantification of green signal (PHKH67) per number of nuclei was used to determine fluorescence. Nuclei were calculated with Fiji by the default auto threshold segmentation method and watershed separation following primary smoothing or were manually counted if the separation of cells was not achieved. Small objects, not of nuclei body size, were removed.

### Sex determination

Determinations of rabbit sex were performed by confirming the presence of the sex-determining region Y gene (gene ID: 100,328,958) in the rabbit genome using PCR and gel electrophoresis visualization as described in A. Hellström et al., Insulin-like growth factor 1 has multisystem effects on foetal and preterm infant development. Acta Paediatr 105, 576-586 (2016). Briefly, DNA was extracted from an ear biopsy using DNeasy Blood and Tissue Kit (Qiagen) according to the manufacturer's instructions. One µl of DNA (range: 100-200 ng/µl) was used in the PCR reaction (30 cyclesat 57 °C) with the following primers: Sense: TGCAATACAGGAGGAACACG, Antisense: AGCAAACTGTCGCTCTTCTG. Presence of a band at approximately 299 bp determined the animal as male, and no corresponding band determined the animal as female.

### Statistics

Statistical significance was calculated using one-way ANOVA with a post hoc Tukey for multiple comparisons of means, or Student's t-test for pair-wise comparisons. P-values < 0.05 were considered significant. Data is presented as means ± SD. Statistical analyses were performed using R version 4.3.1.

### Results

**Table 7 Top 20 of the top 100 proteins that are often identified in EVs (http://microvesicles.org/index.html)**

| **Preterm piglet CSF EVs** | **ChPE EVs** | **Annotation** | **Numbers of times identified** |
|---|---|---|---|
| PDCD6IP | PDCD6IP | Programmed cell death 6-interacting protein | 399 |
| GAPDH | GAPDH | Glyceraldehyde-3-phosphate dehydrogenase | 377 |
| HSPA8 | HSPA8 | Heat shock cognate 71 kDa protein | 363 |
| ANXA2 | ANXA2 | Annexin A2 | 350 |
| CD9 | | CD9 antigen | 337 |
| PKM | PKM | Pyruvate kinase PKM | 328 |
| HSP90AA1 | HSP90AA1 | Heat shock protein HSP 90-alpha | 327 |
| ENO1 | ENO1 | Alpha-enolase | 327 |
| ANXA5 | ANXA5 | Annexin A5 | 327 |
| HSP90AB1 | HSP90AB1 | Heat shock protein HSP 90-beta | 313 |
| | CD63 | CD63 antigen | 306 |
| YWHAZ | YWHAZ | 14-3-3 protein zeta/delta | 306 |
| | YWHAE | 14-3-3 protein epsilon | 301 |
| EEF1A1 | EEF1A1 | Elongation factor 1-alpha 1 | 300 |
| PGK1 | PGK1 | Phosphoglycerate kinase 1 | 295 |
| CLTC | CLTC | Clathrin heavy chain 1 | 291 |
| PPIA | PPIA | Peptidyl-prolyl cis-trans isomerase A, terminally processed N- | 283 |
| SDCBP | SDCBP | Syntenin-1 | 278 |
| ALDOA | ALDOA | Fructose-bisphosphate aldolase A | 277 |

### IGF-1 is localized in intracellular vesicles in a neonatal primary murine ChPE transwell cell culture model

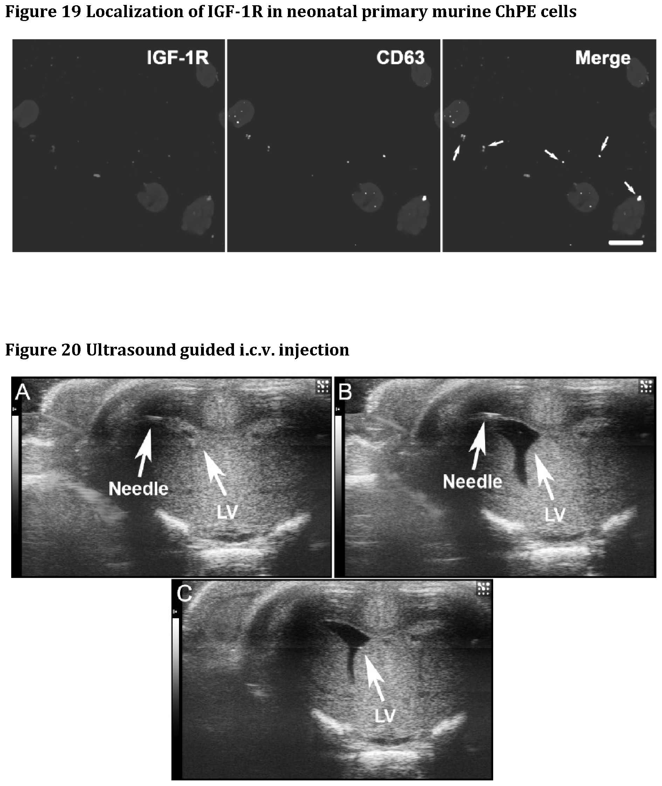

To characterize how circulating human IGF-1 (hIGF-1) affects neonatal ChPE cell secretion of EVs, and its content, we established a transwell in vitro cell culture system using postnatal day 3-8 primary murine ChPE cells. In this system, exposure from the circulation can be mimicked from the basal side, whereas the CSF-side is represented by the apical side, see schematic illustration in Fig.12 A. The cells were cultured as describe previously (T. R. Menheniott, M. Charalambous, A. Ward, Derivation of primary choroid plexus epithelial cells from the mouse. Methods Mol Biol 633, 207-220 (2010), and the model was characterized by verifying positive expression of the ChPE marker transthyretin (TTR, green) and the tight junction protein zonula occludens-1 (ZO-1, red) (Table 7, Fig. 12 A and B, respectively) (I. Kratzer, J. Ek, H. Stolp, The molecular anatomy and functions of the choroid plexus in healthy and diseased brain. Biochimica et Biophysica Acta (BBA) - Biomembranes 1862, 183430 (2020)). Additionally, absence of fibroblast contamination was confirmed by labeling with S100A4 and heat shock protein (Hsp) 47 (Table 7, Fig. 12C and D) (183430 (2020).F. Strutz et al., Identification and characterization of a fibroblast marker: FSP1. J Cell Biol 130, 393-405 (1995), T. Miyamura et al., Small molecule inhibitor of HSP47 prevents pro-fibrotic mechanisms of fibroblasts in vitro. Biochemical and Biophysical Research Communications 530, 561-565 (2020). Moreover, the formation of a blood-CSF-barrier was confirmed by transepithelial electrical resistance (TEER) measurements and conducting a transcytosis assay (Table 7, Fig. S12 E and F) (B. Srinivasan et al., TEER measurement techniques for in vitro barrier model systems. J Lab Autom 20, 107-126 (2015)). Following exposure to 40 ng/ml hIGF-1 on the basal side for 24 hours, a presence of IGF-1 was observed within the cultured ChPE cells using confocal light microscopy (Fig. 12 B middle-left). Furthermore, labeling against IGF-1R also displayed positive signals within the ChPE cells (S1 Appendix, Fig. 19). Interestingly, co-labeling with the late endosomal marker CD63, characteristic for multivesicular bodies (Z. Andreu, M. Yáñez-Mó, Tetraspanins in extracellular vesicle formation and function. Front Immunol 5, 442 (2014).), displayed co-localization with both IGF-1, IGF-1R and TTR, indicating intracellular vesicle location of both IGF-1, its receptor and TTR (Fig. 12 B far right and Table 7, Fig. 19). Moreover, TEM immunogold labeling of the exosomal marker flotillin-2, displayed presence of intracellular membrane enclosed vesicles (Fig. 12 C, white arrows), indicating production of EVs (20). In addition, immunogold labeling against IGF-1 revealed an accumulation in intracellular membrane enclosed vesicles (Fig. 12 D and E, white arrows). An accumulation of IGF-1 was also visible in the ChPE mitochondria (Fig. 12 F), and TEM-analysis revealed presence of IGF-1 in membrane budding in the ChPE cells (Fig. 12 G) and an indication of IGF-1 positive vesicles release into the extracellular space (Fig. 12 G and H, white arrows). Of note, it was not feasible to determine if the IGF-1 retained within the vesicle was hIGF-1 or murine IGF-1 as the antibody used detects both epitopes.

### ChPE cells secrete IGF-1 positive EVs upon hIGF-1 stimulation

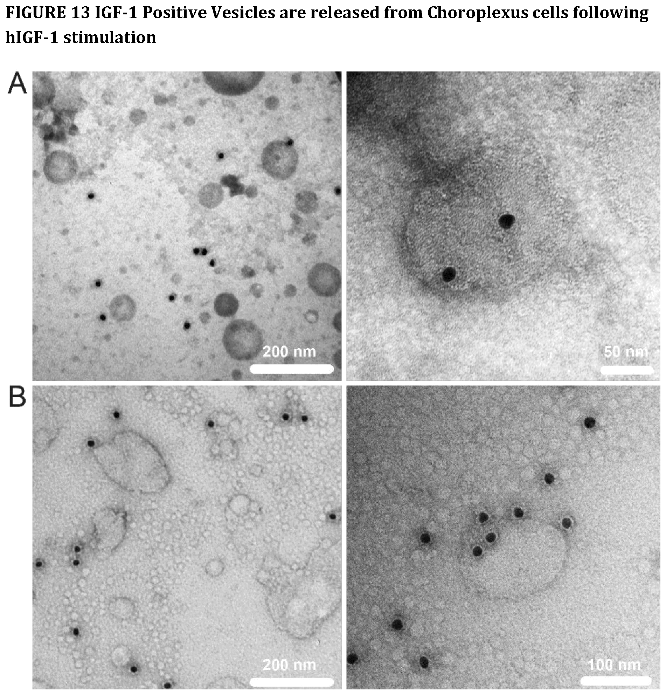

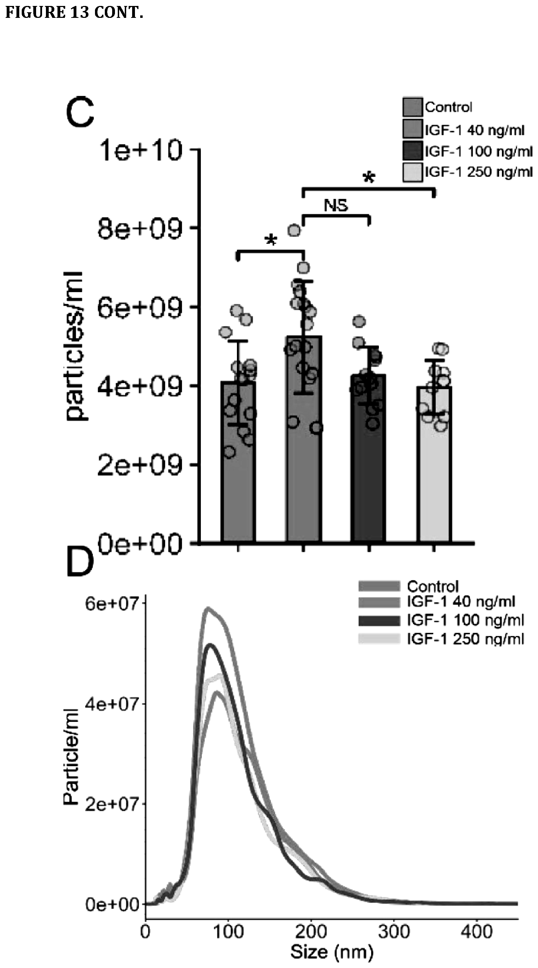

Next, we aimed to evaluate if hIGF-1 is transported from the ChPE cells in intracellular vesicles to the extracellular space. The neonatal primary murine ChPE cells were exposed to hIGF-1 (40, 100 and 250 ng/ml) at the basal side for 24 hours, and the supernatant at the apical side was collected for subsequent EV preparation and analysis. Successful preparation of EVs were verified by TEM analysis, and confirmed presence of exosomes, by showing positive labeling for the exosomal marker flotillin-2, in the ChPE cell secreted supernatant (Fig. 13 A). TEM analysis of IGF-1, using immunogold labeling, displayed presence on/in the purified vesicles, both larger and smaller vesicles (Fig. 13 B). Of note, separation of hIGF-1 and murine IGF-1 was not feasible as the antibody used detects both epitopes. Subsequently, we analyzed the amount of EVs released into the apical supernatant, measured by nanoparticle tracking analysis (NTA), following exposure of ChPE cells to 40-250 ng/ml of hIGF-1. Exposure to 40 ng/ml hIGF-1, but not 100 and 250 ng/ml, displayed a significant increase of ChPE cell released EVs as compared to Control (Fig. 13C and D). Based on these results, 40 ng/ml hIGF-1 was used in subsequent experiments.

### Proteomic analysis reveals presence of hIGF-1 in ChPE derived EVs upon hIGF-1 stimulation

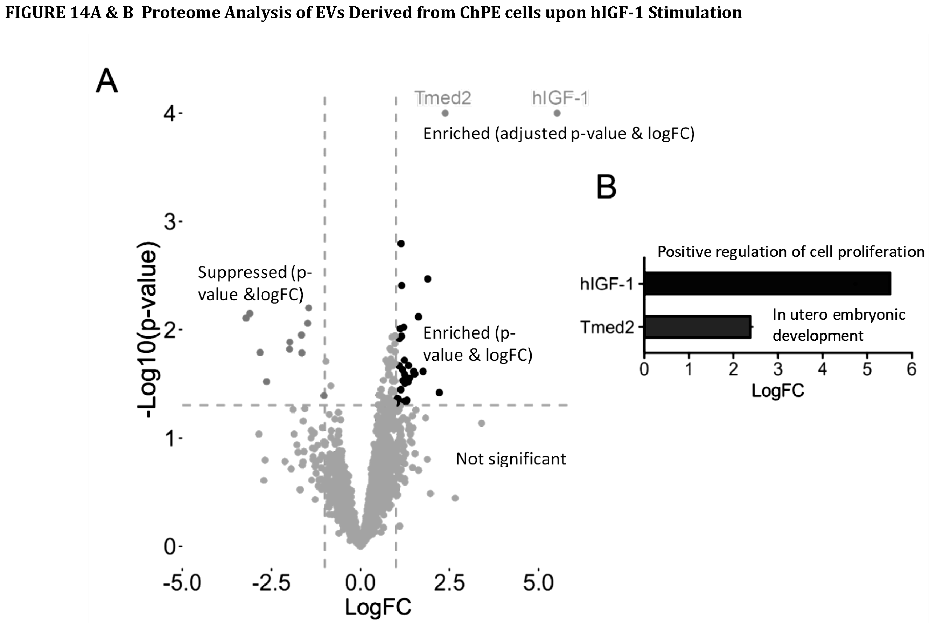

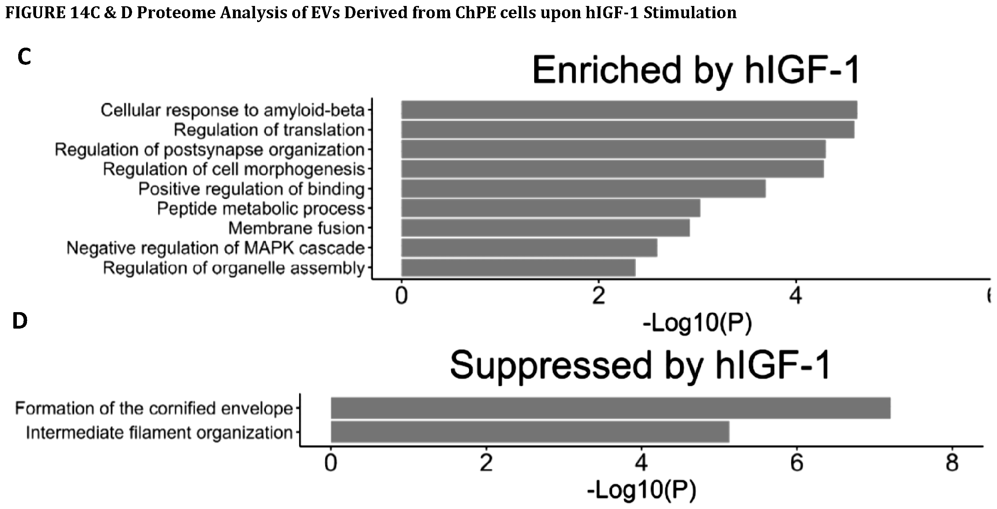

Next, we asked if the proteome, obtained by mass spectrometry (MS) analysis, of EVs, purified from ChPE cell supernatant, would alter following hIGF-1 exposure. Overall, hIGF-1 induced marginal effects when the significant criteria was set to adjusted p-value (Benjamini-Hochberg-corrected p-value ≤ 0.05) (Fig. 14 A). Interestingly, hIGF-1 along with Transmembrane P24 Trafficking Protein 2 (Tmed2), important for in utero embryonic development (R. Aber, W. Chan, S. Mugisha, L. A. Jerome-Majewska, Transmembrane emp24 domain proteins in development and disease. Genet Res (Camb) 101, e14 (2019), were significantly enriched (Fig. 14 A, yellow dots, and B). Next, we submitted proteins with less strict significant criteria (log fold-change ≥ 1 and ≤ -1, and p-value ≤ 0.05) (Fig. 14 A, red and blue dots) to Metascape. The top 4 output displayed enrichment of pathways involved in cellular response to amyloid-beta, regulation of translation, regulation of post-synapse organization, and regulation of cell morphogenesis following hIGF-1 stimulation (Fig. 14 C). Suppressed pathways in ChPE cells derived EVs upon hIGF-1 stimulation were formation of the cornified envelope and intermediate filament organization (Fig. 14 D).

### EV proteomes are similar between ChPE supernatant and preterm piglet CSF

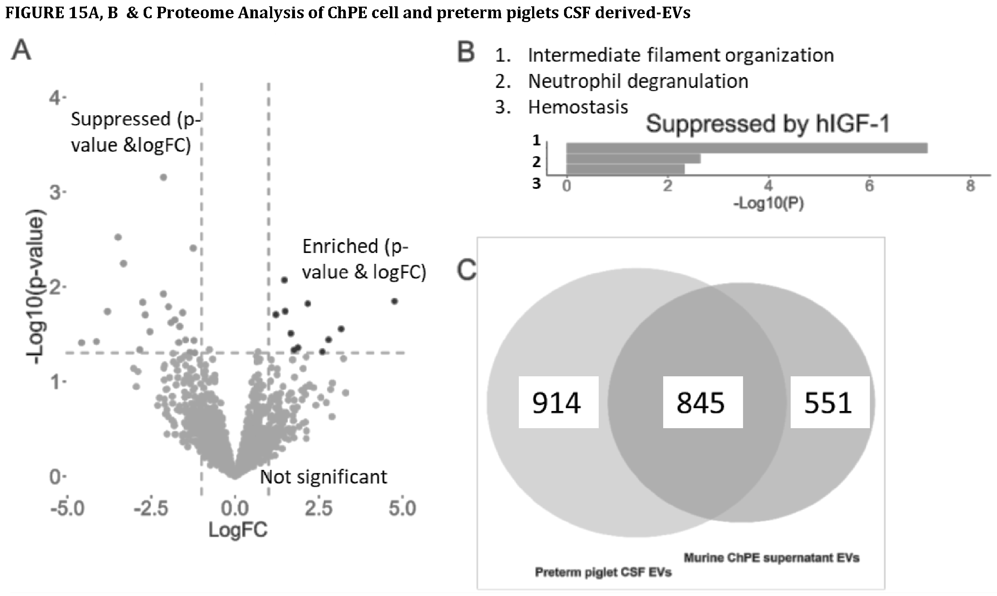

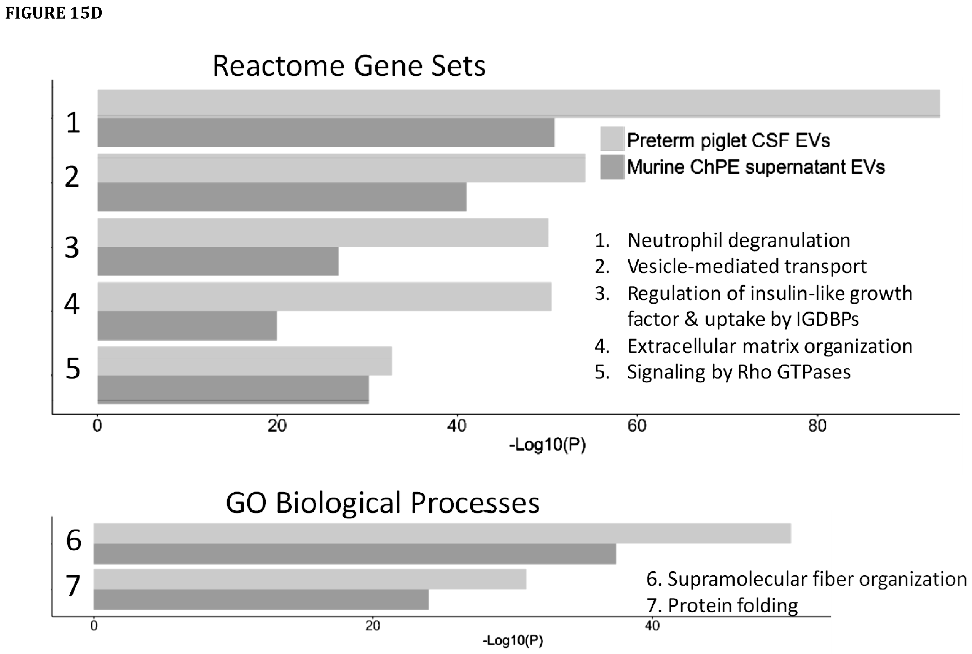

To expand our understanding of the impact of systemic hIGF-1 on the ChP secreted EVs, we investigated if the hIGF-1 exposed ChPE cell derived EV proteome share similarities with that of EVs derived from preterm piglets CSF. Preterm piglets, delivered by cesarian section at a gestational age of 106 days (full term = 117 days), were exposed to recombinant hIGF-1 (in complex with IGF-1 binding protein 3 and henceforth referred to as hIGF-1, 2.25 mg/kg/day via a continuous infusion), or the corresponding vehicle solution (Control group) for 9 days, and CSF were collected at termination. Analysis of the proteome, using MS-analysis with significance criteria set to adjusted p-value (Benjamini-Hochberg-corrected p-value ≤ 0.05), displayed a similar pattern as the ChPE cell derived EVs with only minor changes in the proteome following hIGF-1 exposure (Fig. 15 A). In line with ChPE cell derived EVs, submitting proteins with less strict significance criteria (log fold-change ≥ 1 and ≤ -1, and p-value ≤ 0.05) to Metascape identified additional pathways, including a suppression of intermediate filament organization upon exposure to hIGF-1 (Fig. 15 B), further supporting the similarities between the two proteome data sets. Of note, no enriched pathways were found in EVs derived from preterm piglets CSF upon IGF-1 treatment. Inspecting the total proteome of all conditions comparing proteins identified in ChPE cells supernatant derived EVs (1396 unique proteins), and preterm piglets CSF derived EVs (1759 unique proteins), we observed a 50-60 % overlap between the proteomes (Fig 4 C). To identify the pathways enriched in the proteome of the EV samples, we performed a pathway analysis using Metascape (Y. Zhou et al., Metascape provides a biologist-oriented resource for the analysis of systems-level datasets. Nat Commun 10, 1523 (2019). Of the top 20 enriched pathways, 5 of the Reactome gene sets (M. Gillespie et al., The reactome pathway knowledgebase 2022. Nucleic Acids Research 50, D687-D692 (2021).) and 2 of the GO biological process (M. Ashburner et al., Gene ontology: tool for the unification of biology. The Gene Ontology Consortium. Nat Genet 25, 25-29 (2000)) were similarly enriched comparing the two data sets (Fig. 4 D). One of the top enriched Reactome pathways for both data sets were Vesicle-mediated transport (Fig.4 D and 3 B), and 17-18 of the top 20 most common EV markers, published on the Vesiclepedia website (http://microvesicles.org/index.html) (H. Kalra et al., Vesiclepedia: a compendium for extracellular vesicles with continuous community annotation. PLoS Biol 10, e1001450 (2012) & M. Pathan et al., Vesiclepedia 2019: a compendium of RNA, proteins, lipids and metabolites in extracellular vesicles. Nucleic Acids Res 47, D516-d519 (2019), were identified in both data sets (Fig 18, Table 7), indicating successful EV preparation from the ChPE supernatant and preterm piglet CSF.

### ChPE derived EVs penetrates into the hippocampus of the preterm rabbit brain

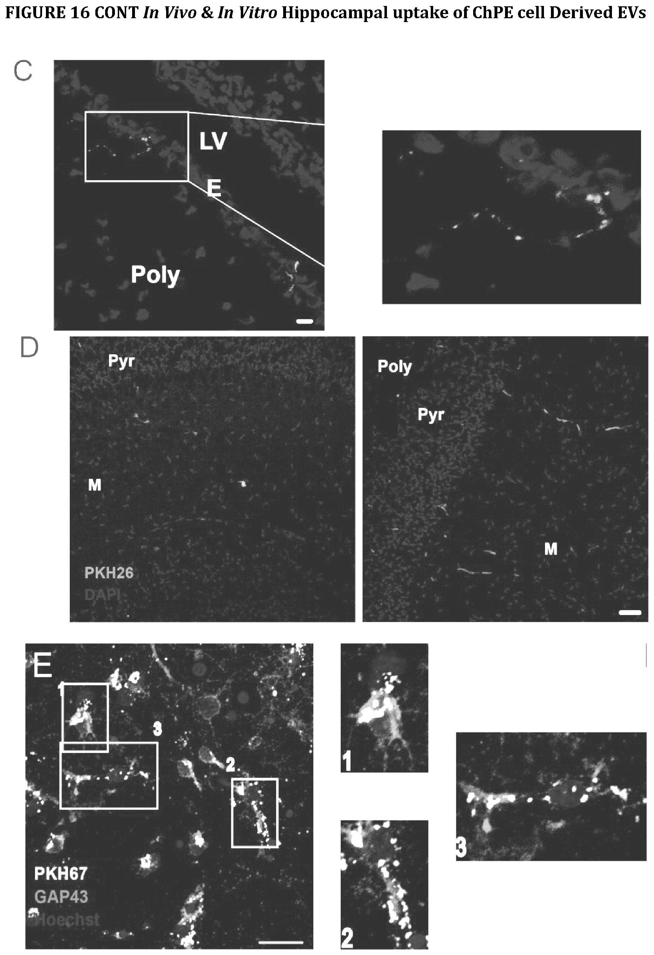

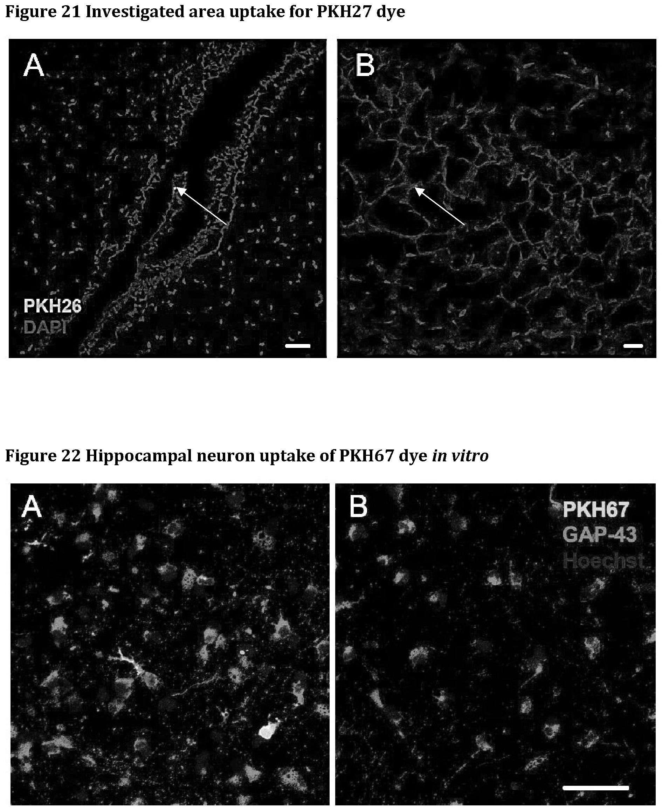

We aimed to investigate if there is a delivery of hIGF-1 stimulated ChPE derived EVs into a targeted subcortical structure. Thus, utilizing a preterm rabbit pup model, we investigated the distribution of purified and labeled ChPE cell derived EVs in the immature brain following intracerebroventricular (i.c.v.) injection. EVs prepared from hIGF-1 stimulated or control neonatal primary murine ChPE cells in vitro were stained with PKH26, and subsequently i.c.v. injected (ultrasound guided) into the lateral ventricles of non-sedated preterm rabbit pups (Table 7, Fig. 20). Pups were terminated 4.5 hours later, and brains were collected and analyzed with confocal microscopy, schematic illustration of the experimental outline presented in Fig. 16A. The analysis showed presence of vesicle or organelle-like structures, corresponding to EVs beyond the ependymal lining of the hippocampus (Fig. 16 B and C). In addition, EVs were shown to penetrate deeper into the hippocampus, reaching the pyramidal and the molecular levels in the cornu ammonis (CA)2-CA3 region (Fig. 16 D). No difference was found in EV uptake comparing EVs derived from hIGF-1 stimulated ChPE cells or control cells (data not shown). Injection of only PKH26 dye resulted in negligible signal in the area of investigation (Table 7, Fig 21).

### ChPE cell derived EVs are internalized by hippocampal neurons in vitro

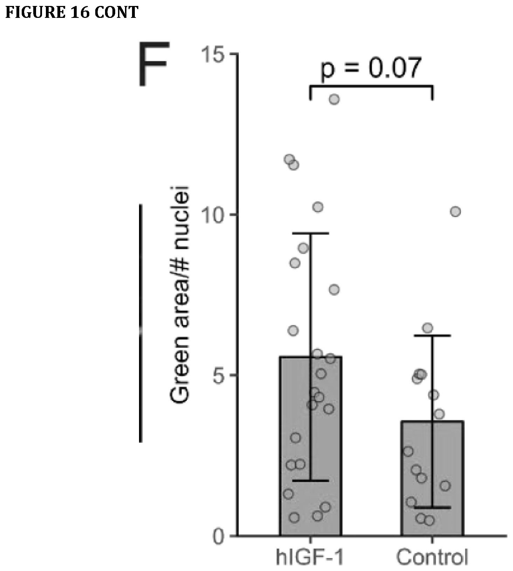

To further characterize the EV mediated interaction between the ChP and the hippocampus, we performed an EV uptake experiment in vitro. Primary rat hippocampal neurons were incubated with purified neonatal primary murine ChPE cell derived EVs labeled with the membrane label PKH67 (green). Following exposure for two hours, the neurons were fixed, labeled with GAP-43 (red) and analyzed by confocal microscopy, see figure 16 A. Interestingly, EVs were observed to be readily taken up in the cytoplasm and in the neurites (Fig. 16 E). Furthermore, comparing exposure of neurons to hIGF-1 stimulated ChPE cell EVs vs. EVs from unstimulated control ChPE cells, we observed a trend (p = 0.07) towards an increased uptake following hIGF-1 stimulation, (Fig. 16 F). Hippocampal neurons exposed to only PKH67 dye showed a dye uptake, however very distinct from neurons exposed to stained EVs (Table 7, Fig. 22 A) and no PKH67 signal was detected in neurons exposed to PBS (Table 7, Fig. 22B).

### Discussion

The ChP is regarded as the gateway to the brain. Here, we describe the ChP as a passage for blood born IGF-1 into deep parts of parenchyma of the developing brain, including the hippocampus. We show that the transportation of IGF-1 appears to be via ChPE-derived EVs. In addition, our experiments indicate that IGF-1 influences the secretion of ChPE-derived EVs, both increasing the amount of EVs secreted, as well as the cargo carried.

The receptor of growth factor IGF-1 is abundantly expressed across the ChP, providing a possible ligand-receptor interaction of IGF-1 at the blood-CSF-barrier that enables interaction both from the CSF and the blood side. Interestingly, it was shown by Pulford et al. that the uptake of IGF-1 from circulation is non-IGF-1R or binding protein dependent, and we have observed an abundance of the IGF-1R on the CSF facing side of the ChP rather than the blood side. Consequently, we hypothesized that the transport of IGF-1 from the circulation to the immature brain may involve uptake of IGF-1 via endocytosis in the ChP, and a subsequent EV secretion into the CSF. The transport of IGF-1 from the blood to the brain, via interaction with the ChP, has been studied previously. For instance, Carro et al. showed that the ChP is the main route of uptake from blood to the parenchyma in an adult mice exercise model. However, to the best of our knowledge, our study is the first that investigates the effects of IGF-1 on EV formation and secretion from the ChP in the context of the immature brain. By stimulating the ChPE cells in our transwell in vitro model with IGF-1 on the basal side i.e. corresponding to the blood side, we observed an uptake and accumulation in membrane enclosed vesicles of IGF-1 in the ChPE cells. We further observed a translocation of IGF-1 through the ChPE via intracellular vesicles to the apical supernatant that was followed by a secretion of IGF-1 positive EVs into the apical culture medium, i.e. corresponding to the CSF. Interestingly, we also observed that intracellular vesicles carrying IGF-1, displayed positive immunolabeling for the IGF-1R, indicating packaging of IGF-1 may occur together with its receptor. To investigate the relevance of the relevance of the in vitro EV-IGF-1 transportation findings, we characterized the proteome content of the ChPE cell derived EVs and compared it to that of the proteome of preterm piglets CSF derived EVs, following systemic exposure to hIGF-1. Encouragingly, the analysis displayed large similarities between the two data sets, including that 60 % of the proteomes were identical, and the pathway of vesicle mediated transport were enriched in both data sets. Although globally we observed only minor differences in the proteome of both data sets, when comparing hIGF-1 stimulation vs. non stimulated one major finding was that hIGF-1 was enriched in EVs derived from hIGF-1 stimulated ChPE cells, thus proving further evidence of IGF-1 transport in EVs through the blood-CSF barrier.

Based on the observation that IGF-1 encapsulated EVs are secreted into the apical supernatant, i.e. into a compartment corresponding to the CSF, we hypothesized that they are destined for transportation of IGF-1 to recipient cells beyond the ventricular ependyma. Previous studies have in fact shown that ChP derived EVs penetrate the ependymal lining of the ventricles followed by an uptake in astrocytes and microglia. For instance, Grapp et al. and Balusu et al. showed that in a murine adult brain, the ChPE secretes EVs into the CSF which penetrates the ependymal protective cells of the parenchyma, thus enabling delivery of molecules from the ChP to the brain. Grapp et al. further showed how the secretion of EVs from the ChPE to the CNS provides nutrients from the blood to the CNS. In addition, Balusu et al. showed that certain systemic stimuli, such as inflammation, enhanced the EV formation and secretion from the ChPE. However, to the best of our knowledge, a potential distribution of the ChPE-derived EVs to a specific subcortical structure has not been investigated. Importantly, previous studies have, as far as we know, utilized adult murine models to investigate the ChP derived EV penetration of the brain parenchyma. In addition, previous studies investigating distribution of intraventricularly injected EVs have used sedated animals. Sedation is, however, well known to significantly affects circulation and perfusion of the brain. In this study, we therefore performed i.c.v. injection ultrasound guided, of ChPE derived EVs in non-sedated preterm rabbit pups, a model highly suitable and widely used for studies of the immature brain. Surprisingly, we found presence of EVs deep into the hippocampus, and to a certain extent in the deeper layers of the pyramidal and molecular layer of the CA2-CA3 region. To further substantiate and characterize this finding, we investigated the ability of primary hippocampal neurons to take up purified ChPE derived EVs in an in vitro setting. Interestingly, hippocampal neurons displayed an extensive uptake of EVs purified from ChPE cells, and EVs were observed both in the cytoplasm and in the neurites. Altogether, this might suggest a targeted delivery of the ChP derived EVs to the hippocampus of the immature brain. The effect of this delivery has not been studied here, however, considering the change in proteome of the ChP derived EVs following IGF-1 exposure, it might be speculated that it could induce enhanced hippocampal neurodevelopment. In fact, Christiansen et al recently observed that preterm piglets exposed to systemic IGF-1 displayed an increased hippocampus neurodevelopment. Moreover, preterm birth causes delayed maturation of the hippocampus in preterm rabbit pups. This clearly warrants further studies on the impact and relevance of IGF-1 mediated communication between ChP and the developing brain.

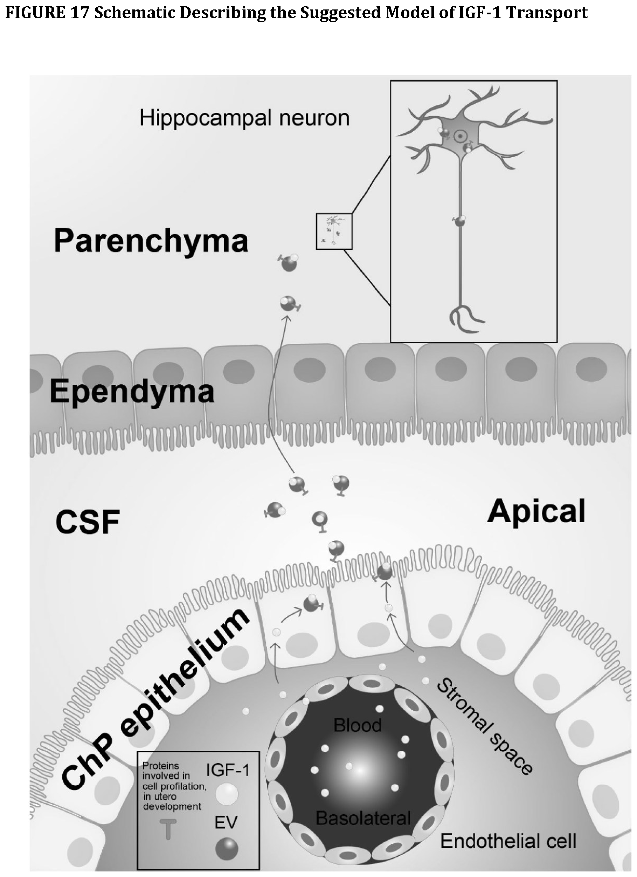

In summary, this study suggests a mechanism for an EV-encapsulated IGF-1 transportation through the blood-CSF barrier and beyond the ependymal lining into the hippocampus in the developing brain (as depicted in Fig. 17). The functions of EV mediated communication from the ChP to the surrounding parenchyma requires more research as this messenger system may provide new therapeutic opportunities for supporting neurodevelopment following EPT birth.

### Numbered Paragraphs:

1. A method of preventing intraventricular haemorrhage (IVH) in a preterm infant born at a low gestational age (for example 23, 24, 25 or 26 weeks, such as 23, 24 or 25) by administering a therapeutic amount of a composition comprising IGF-1 and an IGF binding protein (such as IGFBP-3), for example as complex, at a dose of 200-500µg/Kg/day (such as 250, 300, 350, 400, 450 or 500, in particular 350 to 500µg/Kg/day) within 3 hours of birth for a period of at least 7 days, such that plasma levels of IGF-1 are maintained within the therapeutic range 28µg/L to 109µg/L during the treatment period, for example and wherein after exclusion of baseline assessment timepoint more than 50% of those infants receiving treatment are IVH free.
2. A method according to paragraph 1, wherein 51, 52, 53, 54, 55, 56, 57, 58, 59, 60, 61, 62, 63, 64, 65% or more of the treated patient population are free from IVH.
3. A method according to paragraph 1 or 2, wherein at least 70% of the treated population, for example 71, 72, 73, 74, 75, 76, 77, 78, 79, 80% or more of the treated population is below grade 2 (i.e. 0 or 1) on the IVH scale, for example by VOLPE method and/or maximum score method.
4. A method according to any one of paragraphs 1 to 3, wherein at least 85% of the treated population, for example 85, 86, 87, 87.5% or more of the treated population is below grade 3 (i.e. 0, 1 or 2) on the IVH scale, for example by VOLPE method and/or maximum score method.
5. A method of preventing intraventricular haemorrhage (IVH) grade 3 or 4 in a preterm infant born at a low gestational age (for example 23, 24, 25 or 26 weeks, such as 26 weeks), by administering a therapeutic amount of a composition comprising IGF-1 and an IGF binding protein (such as IGFBP-3), for example as complex, at a dose of 200-500/µg/Kg/day (such as 250, 300, 350, 400, 450 or 500, in particular 350 to 500µg/Kg/day) within 3 hours of birth for a period of at least 7 days, such that plasma levels of IGF-1 are maintained within the therapeutic range 28µg/L to 109µg/L during the treatment period, wherein after exclusion of the baseline timepoint 15% or less of those infants receiving treatment have grade 3 or 4 IVH, such as 12. 5%, 12%, 11%, 10%, 9%, 8%, 7%, 6.5%, 6.25% or less, in particular 10% or less.
6. A method of preventing intraventricular haemorrhage (IVH) or preventing grade 3 or 4 IVH in a preterm infant with an APGAR score below 7 by administering a therapeutic amount of a composition comprising IGF-1 and an IGF binding protein (such as IGFBP-3), for example as complex, at a dose of 200-500µg/Kg/day (such as 250, 300, 350, 400, 450 or 500 in particular 350 to 500µg/Kg/day) within 3 hours of birth for a period of at least 7 days, such that plasma levels of IGF-1 are maintained within the therapeutic range 28µg/L to 109µg/L during the treatment period, and wherein after exclusion of baseline assessment timepoint a higher percentage of infants receiving treatment are IVH free or grade 3 or 4 free, in comparison to comparable untreated preterm infants.
7. A method of stabilising pathological fluctuations in blood osmolarity of in a preterm infant (for example a low gestational age preterm) by administering a therapeutic amount of a composition comprising IGF-1 and an IGF binding protein (such as IGFBP-3), for example as complex, at a dose of 200-500/µg/Kg/day (such as 250, 300, 350, 400, 450 or 500 in particular 350 to 500µg/Kg/day) within 3 hours of birth for a period of at least 7 days, such that plasma levels of IGF-1 are maintained within the therapeutic range 28µg/L to 109µg/L during the treatment period.
8. A method according to any preceding paragraph, wherein the incidences of IVH occurring and/or progressing are ameliorated in the time period 24 hours to 168 hours after initiations of treatment, such as 24 hours to 120 hours, including 48 to 120 hours.
9. A method according to any one of the preceding paragraphs wherein a treated preterm has a mortality benefit.
10. A method according to any one of the preceding paragraphs wherein the risk of intraventricular haemorrhage (IVH) is reduced by at least 10%, for example 11, 12, 13, 14, 15, 16, 17, 18% or more at 48 hours (such as at least 50, 60 or 72 hours).
11. A method according to any one of the preceding paragraphs, wherein the severity of intraventricular haemorrhage (IVH) is reduced, for example incidence of grade 3 and/or 4 is reduced.
12. A method according to any one of the preceding paragraphs, wherein the severity of IVH grade 2 is reduced.
13. A method according to any preceding paragraphs wherein treatment is initiated within 24 hours from birth, for example within 30 mins to 3 hours from birth.
14. A method according to any preceding paragraph, wherein the treatment is administered subcutaneously and/or by infusion (such as intermittent or continuous infusion).
15. A method according to any preceding paragraph, wherein the preterm infants are treated for at least 5 days, for example for at least 1 week, such as 2 to 6 weeks, such as 2, 3, 4, 5 or 6 weeks.
16. A method according to any preceding paragraph, wherein treatment is continued until 32, 33 or 34 weeks gestational age.
17. A method according to any preceding paragraph, wherein serum levels of IGF-1 are maintained within the range 28 to 109ng/mL.

## Claims

1. A composition comprising IGF-1 and IGFBP-3 for use in treating a preterm neonate by administering a therapeutic amount of a dose in the range 350 to 500µg/Kg/day to stabilising (one or more) vital functions and provide a reduction in mortality benefit.

2. A composition according to claim 1 wherein the preterm neonate was born extremely premature.

3. A composition according to claim 1 or 2, wherein the premature neonate was born at a low gestational age.

4. A composition according to any one of claims 1 to 3, wherein the instability is selected from blood pressure, respiratory and kidney function problems.

5. A composition according to any one of claims 1 to 4, wherein treated neonates are more robust.

6. A composition according to claim 5 wherein more robustness is selected from blood pressure stabilisation and ability to urinate stabilisation.

7. A composition according to any one of claims 1 to 6 wherein the neonate a low APGAR score.

8. A composition according to any one of claims 1 to 7, wherein the treatment protects from circulatory collapse.

9. A composition according to any one of claims 1 to 8, wherein the treatment stabilises vital functions when hyperosmolarity occurs.

10. A composition according to any one of claims 1 to 9, wherein the treatment stabilises acute metabolic syndrome.

11. A composition according to any one of claims 1 to 10, wherein treatment helps stabilise fluctuations in hyperosmolarity.
